# EUROPÄISCHE PATENTSCHRIFT

(11) **EP 3 700 884 B1**
(45) Veröffentlichungstag und Bekanntmachung des Hinweises auf die Patenterteilung: **13.10.2021**
(21) Anmeldenummer: 18785386.6
(22) Anmeldetag: 17.10.2018
(51) Int. Cl.: C07C 209/16, C07C 213/02, C07C 211/10

(54) **VERFAHREN ZUR HERSTELLUNG VON ETHYLENAMINEN**
PROCESS FOR THE PREPARATION OF ETHYLENEAMINES
PROCEDE DE PRODUCTION D'ETHYLENEAMINES

(30) Priorität: 27.10.2017 EP 17198935
(43) Veröffentlichungstag der Anmeldung: 02.09.2020
(73) Patentinhaber: BASF SE, 67056 Ludwigshafen am Rhein (DE)
(72) Erfinder: LUYKEN, Hermann, 67056 Ludwigshafen (DE)
(74) Vertreter: BASF IP Association
(86) Internationale Anmeldenummer: PCT/EP2018/078324
(87) Internationale Veröffentlichungsnummer: WO 2019/081285

(56) Entgegenhaltungen:
- WO-A1-2005/037769
- WO-A1-2007/093514
- WO-A1-2007/093552

## Beschreibung

Die vorliegende Erfindung betrifft ein Verfahren zur Herstellung von Ethanolaminen und/oder Ethylenaminen ausgehend von Monoethylenglykol.

Zur großtechnischen Herstellung von Ethylendiamin (EDA) kommen in der Regel zwei Verfahren zur Anwendung.

Zum einen kann EDA durch Umsetzung 1,2 Dichlorethan mit Ammoniak unter Abspaltung von HCI hergestellt werden (EDC-Verfahren). Ein weiteres großtechnisches Verfahren zur Herstellung von EDA ist die Umsetzung von Monoethanolamin (MEA) mit Ammoniak in Gegenwart von Aminierungskatalysatoren (MEA-Verfahren).

Alternativ zu den etablierten Verfahren kann die Herstellung von EDA auch durch Umsetzung von Monoethylenglykol (MEG) mit Ammoniak erfolgen.
Ein solches Verfahren hätte verschiedene Vorteile. Ein Vorteil besteht in der guten Verfügbarkeit von MEG im Vergleich zu MEA.
MEA wird großtechnisch durch Umsetzung von Ethylenoxid (EO) und Ammoniak hergestellt. Es entsteht in der Regel ein Reaktionsgemisch, welches neben MEA auch noch höhere Ethanolamine, wie Diethanolamin (DEOA) und Triethanolamin (TEOA) enthält. Diese Nebenprodukte müssen von MEA durch einen separaten Destillationsschritt abgetrennt werden. Ethylenoxid ist ein hochentzündliches Gas, welches mit Luft explosionsfähige Gemische bilden kann. Die Handhabung von EO ist entsprechend aufwendig. Die Herstellung von MEA erfordert somit eine technisch aufwendige EO-Anlage mit anschließender Reindestillation.
Demgegenüber kann MEG sowohl auf Basis von petrochemischen Rohstoffen als auch auf Basis von nachwachsenden Rohstoffen produziert werden. Petrochemisch wird MEG ebenfalls aus EO durch Umsetzung mit Wasser hergestellt. Ebenso wie bei der Umsetzung von EO mit Ammoniak, lässt sich bei der Umsetzung von EO mit Wasser nicht verhindern, dass bereits entstandenes MEG mit EO zu Nebenprodukten, wie Di- und Triethylenglykol, reagieren kann. Die Selektivität von MEG liegt bei ca. 90% und ist somit aber deutlich höher als die Selektivität von MEA, welche in der Regel bei 70-80% liegt. Durch den Omega-Prozess der Schell konnte die Selektivität für MEG jedoch nochmals deutlich - auf ca. 99% -- gesteigert werden. Im Omega-Prozess wird EO mit CO₂ zu Ethylencarbonat umgesetzt, welches im zweiten Schritt selektiv zu MEG hydrolysiert wird.
MEG lässt sich auch über den Synthesegasweg, z.B. durch oxidative Carbonylierung von Methanol zu Dimethyloxalat und dessen anschließender Hydrierung, herstellen. Damit kommen als weiterer petrochemischer Rohstoff auch Erdgas oder Kohle für die Herstellung von MEG in Frage.
Alternativ, kann MEG auch aus nachwachsenden Rohstoffen, wie Mais oder Zuckerrohr durch Fermentation zu Ethanol, anschließender Dehydratisierung zu Ethen und nachfolgender Umsetzung mit Sauerstoff zu Ethylenoxid hergestellt werden.

Aufgrund der vielen Herstellungsvarianten ist die Verfügbarkeit von MEG im Allgemeinen hoch, was sich in der Regel positiv auf die Rohstoffkosten auswirkt.

Im Stand der Technik wird offenbart, dass die Umsetzung von MEG mit Ammoniak zu EDA sowohl in der Flüssigphase als auch in der Gasphase erfolgen kann.

Die Aminierung von MEG in der Gasphase wird in den beiden chinesischen Anmeldungen CN 102 190 588 und CN 102 233 272 offenbart.
So beschreibt die CN 102 190 588 die einstufige Umsetzung von MEG und Ammoniak in Gegenwart von Cu-haltigen Katalysatoren. Der Reaktionsdruck liegt gemäß der Beschreibung in einem Bereich von 3 bis 30 bar. Die Reaktionstemperatur liegt im Bereich von 150 bis 350°C.
In der Anmeldung CN 102 233 272 wird die Umsetzung von MEG mit Ammoniak in der Gasphase an Katalysatoren offenbart, die Cu und Ni als Hauptbestandteile und Zr, Zn, Al, Ti, Mn und Ce als Nebenkomponente beinhalten. Die Zusammensetzung der erhaltenen Reaktionsgemische wurde allerdings nicht offenbart.

Alternativ zur Umsetzung in der Gasphase kann die Umsetzung von MEG mit Ammoniak und Wasserstoff auch in der Flüssigphase erfolgen. Das Reaktionsverhalten von Katalysatoren in der Gas- und Flüssigphase unterscheidet sich jedoch in der Regel erheblich, so dass Rückschlüsse von dem Reaktionsverhalten von MEG in der Gasphase auf das Reaktionsverhalten von MEG in der Flüssigphase im Allgemeinen nicht zulässig sind.

Eine Übersicht über die metallkatalysierte Aminierung von M EG in der flüssigen Phase wird in der Diplomarbeit "Reaktionskinetische Untersuchungen zur metallkatalysierten Aminierung von Ethylenglykol in der flüssigen Phase" von Carsten Wolfgang Ihmels gegeben ("Reaktionskinetische Untersuchungen zur metallkatalysierten Aminierung von Ethylenglykol in der flüssigen Phase", Diplomarbeit der Carl von Ossietzky Universität Oldenburg vom 17.03.2000). Ihmels beschreibt eine Vielzahl von Folge- und Nebenreaktionen, die bei der Aminierung von MEG auftreten können, beispielsweise die Bildung von Di- und Triethanolamin, Disproportionierung, Nitrilbildung, Carbonylkondensation und Fragmentierungsreaktionen. Kondensation und Dispoportionierung können bei zweiwertigen Alkoholen letztendlich auch zur Bildung von Oligomeren, wie Diethylentriamin (DETA), Triethylentetramin (TETA) und Polymeren führen. Eine weitere wichtige Nebenreaktion ist die Cyclisierung. So kann Diethanolamin oder DETA zum Piperazin (PIP) weiterreagieren. Höhere Temperaturen fördern eine sich an die Cyclisierung anschließende Dehydrierung zu Aromaten. Somit wird bei der Umsetzung von MEG mit Ammoniak ein breites Produktspektrum erhalten, wobei einige Produkte in dem Produktspektrum kommerziell interessanter sind als andere. So ist für EDA, DETA und TETA der kommerzielle Bedarf höher als der von PIP oder Aminoethylethanolamin (AEEA). Gegenstand von vielen Untersuchungen bei der Umsetzung von MEG mit Ammoniak war es deshalb Katalysatoren und Reaktionsbedingungen zu finden, die zu einem vorteilhaften Produktspektrum führen.

US 4,111,840 offenbart die Umsetzung von MEG mit Ammoniak und Wasserstoff bei Drücken von 500 bis 5000 psig (ca. 34 bis 340 bar) an geträgerten Ni/Re-Katalysatoren. Dabei führten Silika/Alumina-Trägerkatalysatoren mit einer Oberfläche von 60 m²/g zu besseren Ergebnissen als Silika/Alumina-Trägerkatalysatoren mit einer spezifischen Oberfläche von 150 m²/g.

In der US 3,137,730 wird die Umsetzung von MEG mit Ammoniak in der Flüssigphase bei Temperaturen von 200-300°C und Drücken oberhalb von 1000 psig (ca. 69 bar) an Cu/Ni-Katalysatoren offenbart.

DE 1 172 268 offenbart die Umsetzung von Ethylenglykol an Katalysatoren, die mindestens eines der Metalle Cu, Ag, Mn, Fe, Ni und Co enthalten. In einem Beispiel wurde MEG mit Ammoniak bei 180°C und einem Druck von 300 bar in Gegenwart von Wasserstoff an einem CoKatalysator umgesetzt.

Die chinesische Anmeldung CN 106607060 A offenbart Katalysatoren zur Aminierung von MEG in flüssiger Phase.

In der WO 2007/093514 wird ein zweistufiges Verfahren zur Herstellung von EDA offenbart, wobei in der ersten Verfahrensstufe die Aminierung an einem Hydroaminierungskatalysator bis zu einem MEA-Umsatz von maximal 40% durchgeführt wird und in der zweiten Verfahrensstufe ein geträgerter Ru/Co-Katalysatorformkörper mit kleiner Geometrie eingesetzt wird und die zweite Stufe bei einer um mindestens 10°C höheren Temperatur als die erste Verfahrensstufe durchgeführt wird.

In der WO 2007/093552 wird ein Verfahren zur Herstellung von Ethylenaminen und Ethanolaminenoffenbart, welches in Gegenwart eines heterogenen Katalysators durchgeführt wird, dessen Aktivmasse Ruthenium und Cobalt und daneben kein weiteres Metall der Gruppe VIII sowie kein Metall der Gruppe IB enthält. Der Katalysator wird in Form von Formkörpern in verschiedenen Geometrien eingesetzt, deren Größe durch einen maximalen Durchmesser definiert ist.

Die in den beschriebenen Verfahren anfallenden Produktströme werden in der Regel zur Reingewinnung von einzelnen Produkten, insbesondere der besonders gewünschten Produkte EDA und DETA, destillativ aufgetrennt.

In der WO 2007/093555 wird offenbart, dass die destillative Aufarbeitung der Reaktionsprodukte aus der MEG-Umsetzung problematisch ist, da MEG und DETA ein Azeotrop bilden, das vom Druck nahezu unabhängig ist und daher durch Druckwechseldestillation nicht aufgetrennt werden kann. Gemäß WO 2007/09355 liegt die azeotrope Zusammensetzung bei ca. 44 Gew.-% MEG und 56 Gew.-% DETA und hat bei 150 mbar einen Siedepunkt von 154°C, gegenüber dem Siedepunkt von reinem MEG von 144°C bzw. von reinem DETA von 142°C, jeweils bei dem oben aufgeführten Druck von 150 mbar. In der WO 2007/093555 wird deshalb ein Verfahren zur destillativen Auftrennung eines Produktstroms aus der MEG-Umsetzung offenbart, bei dem eine Stufe der Auftrennungssequenz als Extraktivdestillation mit Triethylenglykol (TEG) aus selektivem Lösungsmittel für DETA durchgeführt wird. In der Offenbarung wird eine Trennsequenz offenbart die folgenden Schritte beinhaltet:
- Einleitung des Austrags aus der MEG-Umsetzung in eine erste Destillationseinheit K-I und Auftrennung des eingeleiteten Austrags in einen Kopfstrom, enthaltend die Komponenten Ethylendiamin und Piperazin, und einem Sumpfstrom, enthaltend die Komponenten mit einem Siedepunkt größer als der Siedepunkt von Piperazin.
- Einleitung des Sumpfstroms aus Kolonne K-I in eine zweite Destillationskolonne K-II und Auftrennung des zugeführten Sumpfstromes in einen Kopfstrom, enthaltend Monoethylenglykol, Diethylentriamin und Monoethanolamin, sowie einen Sumpfstrom, enthaltend gegenüber Monoethylenglykol und Diethylentriamin schwerer siedende Komponenten.
- Zuführung des Kopfstroms aus Kolonne K-II in eine Extraktivdestillationskolonne K-III, der auf gleicher Trennstufe oder Höhe ein Triethylenglykol als selektives Lösungsmittel für Diethylentriamin zugeführt wird, wobei in der Extraktivdestillationskolonne K-III über Sumpf ein mit Diethylentriamin beladener, das selektive Lösungsmittel Triethylenglykol enthaltender Strom, und über Kopf ein Monoethylenglykol enthaltender, weitgehend von Diethylentriamin freier Strom abgezogen wird.
- Der Sumpfstrom aus der Extraktivdestillationskolonne K-III, enthaltend mit DETA beladenes selektives Lösungsmittel, wird bevorzugt einer Desorptionskolonne K-IV zugeführt, und darin in einen DETA enthaltenden Kopfstrom und einen TEG enthaltenden Sumpfstrom aufgetrennt. Der TEG enthaltende Sumpfstrom aus der Kolonne K-IV wird bevorzugt in die Extraktivdestillationskolonne K-III recycliert.

Aufgabe der vorliegenden Erfindung war es, ein Verfahren zur Herstellung von Ethylenaminen und/oder Ethanolaminen zur Verfügung zu stellen, welches besonders ressourceneffizient in Bezug auf die eingesetzten Ausgangsstoffe, Energie und Verbrauchsmittel, insbesondere Waschflüssigkeiten ist.

Die Aufgabe der vorliegenden Erfindung wurde gelöst durch ein
Verfahren zur Herstellung von Ethylenaminen und/oder Alkanolaminen welches folgende Schritte umfasst:
1) Umsetzung von MEG mit Ammoniak in Gegenwart von Wasserstoff und eines Aminierungskatalysators;
2) Abtrennung von Wasserstoff und Ammoniak aus dem Reaktionsaustrag aus Stufe 1, dadurch gekennzeichnet, dass die Abtrennung von Wasserstoff und Ammoniak in Stufe 2 folgende Schritte umfasst:
   2-1) Auftrennung des Reaktionsaustrags aus Stufe 1 in eine gasförmige Phase, welche Ammoniak und Wasserstoff enthält, und eine flüssige Phase, welche Ethylenamine und/oder Alkanolamine enthält,
   2-2) Überleitung der gasförmigen Phase aus Stufe 2-1) über ein oder mehrere Kondensatoren, wobei eine oder mehrere flüssige Phase erhalten werden, in denen Ammoniak angereichert ist, und eine gasförmige Phase erhalten wird, in der Wasserstoff angereichert ist,
   2-3) Inkontaktbringen der gasförmigen Phase aus Stufe 2-2) mit MEG, so dass eine flüssige Phase, enthaltend MEG und Ammoniak, erhalten wird und eine gasförmige Phase, enthaltend Wasserstoff und optional Ammoniak, erhalten wird.

Das vorliegende Verfahren unterscheidet sich vom Stand der Technik dahingehend, dass die Ammoniakabtrennung neu konzeptioniert wurde, so dass ein besonders ressourceneffizientes Verfahren erzielt werden konnte.

Nachfolgend werden folgende Abkürzungen verwendet:
- AEEA:: Aminoethylethanolamin
- AEP:: Aminoethylpiperazin.
- DETA:: Diethylentriamin.
- EDA:: Ethylendiamin.
- EDC:: Ethylendichlorid.
- HEP:: Hydroxyethylpiperazin.
- HPA:: Heavy Polyamine (Schwere Polyamin).
- MEA:: Monoethanolamin.
- MEG:: Monoethylenglykol
- NMEDA:: N-Methylethylendiamin
- PEHA:: Pentaethylenhexamine.
- PIP:: Piperazin.
- TEPA:: Tetraethylenepentamin.
- TETA:: Triethylenetetramin.

Druckangaben beziehen sich, falls nicht anderweitig spezifiziert, auf die Angabe des absoluten Drucks.

Die Erfindung kann folgendermaßen ausgeführt werden.

### MEG-Umsetzung - Stufe 1)

Erfindungsgemäß wird in einer Stufe 1) MEG in Gegenwart von Ammoniak, Wasserstoff und einem heterogenen Katalysator umgesetzt.

### Edukt-MEG

Als Ethylenglykol wird bevorzugt technisches Ethylenglykol mit einer Reinheit von mindestens 98%, und ganz besonders bevorzugt Ethylenglykol mit einer Reinheit von mindestens 99% und ganz besonders bevorzugt von mindestens 99,5% eingesetzt.
Das in dem Verfahren eingesetzte Ethylenglykol kann aus Ethylen hergestellt werden, welches aus petrochemischen Prozessen erhältlich ist. So wird in der Regel in einer ersten Stufe Ethen zu Ethylenoxid oxidiert, welches nachfolgend mit Wasser zu Ethylenglykol umgesetzt wird. Das erhaltene Ethylenoxid kann aber auch im sogenannten Omega-Prozess mit Carbondioxid zu Ethylencarbonat umgesetzt werden, welches anschließend mit Wasser zu Ethylenglykol zu hydrolisieren kann. Das Omega-Verfahren zeichnet sich durch eine höhere Selektivität für Ethylenglykol aus, da weniger Nebenprodukte, wie Di- und Triethylenglykol entstehen.
Ethen kann aber auch aus nachwachsen Rohstoffen hergestellt werden. So kann Ethen durch Dehydratisierung von Bio-Ethanol gebildet werden.
Ethylenglykol lässt sich auch über den Synthesegasweg, z.B. durch oxidative Carbonylierung von Methanol zu Dimethyloxalat und dessen anschließender Hydrierung, herstellen. Damit kommen als weiterer petrochemischer Rohstoff auch Erdgas oder Kohle für die Herstellung von MEG in Frage.

In das erfindungsgemäße Verfahren kann ein MEG eingesetzt werden, dass keine sogenannte Faserqualität aufweist. In diesem Fall ist es jedoch bevorzugt das MEG zusammen mit dem Gemisch C in Stufe b) (wie nachfolgend beschrieben) einzuleiten.

### Schwefelgehalt im MEG

Bevorzugt enthält das eingesetzte MEG weniger als 100 ppm Schwefel. Besonders bevorzugt enthält das eingesetzte MEG weniger als 20 ppm Schwefel, ganz besonders bevorzugt weniger als 2 ppm Schwefel. Im Idealfall enthält das eingesetzte MEG gar keinen Schwefel. Es ist daher vorteilhaft, das frisch eingesetzte MEG vor Einführung in den Reaktionsteil so aufzuarbeiten, dass eventuell vorhandenes Schwefel abgetrennt wird. Dazu besteht die Möglichkeit, frisches MEG statt direkt in die MEG-Umsetzung (Stufe 1), zunächst in Stufe b) oder Stufe 2 oder Stufe a), bevorzugt Stufe b), einzuleiten und nach der Aufrennung in Stufe b) in die MEG-Umsetzung (Stufe 1) zurückzuführen. Es wurde gefunden, dass durch die Rektifikation in Gegenwart von Aminen nicht nur gegebenenfalls im Frisch-MEG vorhandene Hochsieder abgetrennt werden, sondern auch gegebenenfalls enthaltene Schwefelverbindungen, wie Sulfide, Sulfite, Mercaptane oder Thiole, die durch Reaktion oder Schleppwirkung mit den basischen Aminen Addukte (Hochsieder) bilden. Die voranstehend genannten bevorzugten Ausführungsformen ermöglichen somit auch den Einsatz eines MEG, das keine sogenannte Faserqualität aufweist.

In einer bevorzugten Ausführungsform wird MEG deshalb vor dem Einleiten in Stufe 1) zunächst in die Stufe 2) oder bevorzugt in die Stufe b) eingeleitet und das in Stufe b) abgetrennte Gemisch D, welches das destillierte MEG enthält in Stufe 1) eingeleitet. Die in dieser Ausführungsform beschriebene Kopplung der MEG-Umsetzung (Stufe 1) mit Stufe b) ermöglicht somit den Einsatz von MEG, welches keine Faserqualitität aufweist, da überraschenderweise gefunden wurde, dass der Schwefelgehalt in Stufe b) abgesenkt werden kann.

### Edukt-NH3

Die Umsetzung von Ethylenglykol erfolgt im Allgemeinen in Gegenwart von Ammoniak.

Als Ammoniak kann herkömmlich im Handel erhältliches Ammoniak eingesetzt werden, beispielsweise Ammoniak mit einem Gehalt von mehr 98 Gew.-% Ammoniak, bevorzugt mehr als 99 Gew.-% Ammoniak, bevorzugt mehr als 99,5 Gew.-%, insbesondere mehr als 99,8 Gew.-% Ammoniak.

### Edukt-H2

Das erfindungsgemäße Verfahren erfolgt bevorzugt in Gegenwart von Wasserstoff.

Der Wasserstoff kommt im Allgemeinen technisch rein zum Einsatz. Der Wasserstoff kann auch in Form eines Wasserstoffs enthaltenden Gases, d.h. mit Beimengungen anderer Inertgase, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Katalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

### Aminierungskatalysatoren

Die Umsetzung von MEG und Ammoniak erfolgt im Allgemeinen in Gegenwart von Aminierungskatalysatoren.

Als Aminierungskatalysatoren können alle Katalysatoren eingesetzt werden, die die Umsetzung von MEG zu EDA und/oder MEA bewirken.
Bevorzugt werden Aminierungskatalysatoren eingesetzt, die ein oder mehrere Aktivmetalle der Gruppen 7 und/oder 8 und/oder 9 und/oder 10 und/oder 11 des Periodensystems der Elemente enthalten. Beispiele für solche Aktivmetalle sind Sn, Cu, Co, Ni und/oder Fe, sowie auch Edelmetalle wie Rh, Ir, Ru, Pt, Pd, sowie Re.

Die oben genannten Aktivmetalle können in Form von Metallnetzen oder Gittern oder in Form von Schwamm- oder Skelettkatalysatoren nach Raney in das Verfahren eingesetzt werden.

Die Aminierungskatalysatoren können optional ein oder mehrere Katalysatorzusatzelemente umfassen. Bei den Katalysatorzusatzelementen handelt es sich in der Regel um Metalle oder

Halbmetalle ausgewählt aus den Gruppen 1 bis 6, 12 bis 17 des Periodensystems der Elemente und den Metallen der Seltenen Erden.

Bevorzugte Aktivmetalle sind Co, Cu, Ni, Ru und Re. Ganz besonders bevorzugte Aktivemetalle sind Ru, Co, Cu und Ni.

Bevorzugte Katalysatorzusatzelemente sind Zr, Al, Sb, Sn, Pb, Bi, In, Si, Ga, V, Nb, S, P, B, Cr, W, La, Ce, Y und Hf, besonders bevorzugt Sn, P, Pb, Sb, La, Zr, Si und Al.

Besonders bevorzugt sind Katalysatorvorläufer die ein oder mehrere Aktivmetalle ausgewählt aus der Gruppe bestehend aus Cu, Co, Ni und Ru enthalten und optional das Katalysatorzusatzelement Sn enthalten.

Besonders bevorzugt werden Aminierungskatalysatoren eingesetzt, die durch Reduktion von sogenannten Katalysatorvorläufern hergestellt werden.
Im Katalysatorvorläufer liegen die Aktivmetalle und die Katalysatorzusatzelemente im Allgemeinen in Form ihrer sauerstoffhaltigen Verbindungen vor, beispielsweise als Carbonate, Oxide, Mischoxide bzw. Hydroxide der Aktivmetalle bzw. Katalysatorzusatzelemente.

In der Regel werden die Katalysatorvorläufer durch Inkontaktbringen von löslichen Verbindungen der Aktivmetalle bzw. der Katalysatorzusatzelemente mit einem Trägermaterial (Tränkung/Imprägnierung) hergestellt oder durch Fällung der löslichen Verbindungen mit einem Fällungsmittel, wobei die Fällung in Gegenwart von Trägermaterial (sogenannte Auffällung) oder in Abwesenheit von Trägermaterialien (sogenannte Mischfällung) erfolgen kann. Die getränkten oder aufgefällten Trägermaterialien bzw. die ausgefällten Niederschläge der Aktimetalle bzw. der Katalysatorzusatzelemente werden im Allgemeinen nach einer Trockung durch eine Calcinierung in die sauerstoffhaltigen Verbindungen umgewandelt, wobei die Umwandlung in der Regel durch Entwässerung und/oder Zersetzung erfolgt.

Als Trägermaterial kann das Katalysatorzusatzelement Kohlenstoff, beispielsweise in Form von Graphit, Ruß und/oder Aktivkohle verwendet werden.

Bevorzugte Trägermaterialien sind Oxide der Katalysatorzusatzelemente Al, Ti, Zn, Zr und Si oder Mischungen davon, beispielsweise Aluminiumoxid (gamma, delta, theta, alpha, kappa, chi oder Mischungen daraus), Titandioxid (Anatase, Rutil, Brookit oder Mischungen daraus), Zinkoxid, Zirkondioxid Siliziumdioxid (wie Silica, pyrogenes Siliziumdioxid, Kieselgel oder Silikate), Alumosilicate, Mineralien, wie Hydrotalcit, Chrysotil und Sepiolit.

Besonders bevorzugte Trägermaterialien sind Aluminiumoxid oder Zirkonoxid oder Gemische davon.

Ein besonders bevorzugtes Trägermaterial ist Aluminiumoxid.

Im Anschluss an die Calcinierung erfolgt in der Regel eine Reduktion der Katalysatorvorläufer, wobei der Katalysatorvorläufer in seine katalytisch aktive Form umgewandelt wird.
Der Katalysator kann nach der Reduktion mit einem Sauerstoff enthaltenden Gasstrom, wie Luft oder einem Gemisch von Luft mit Stickstoff, in Kontakt gebracht werden.
Dadurch wird ein passivierter Katalysator erhalten. Der passivierte Katalysator weist im Allgemeinen eine schützende Oxidschicht auf. Durch diese schützende Oxidschicht wird die Handhabung und Lagerung des Katalysators vereinfacht, so dass beispielsweise der Einbau des passivierten Katalysators in den Reaktor vereinfacht wird.
Ein passivierter Katalysator wird bevorzugt vor dem Inkontaktbringen mit den Edukten durch Behandlung des passivierten Katalysators mit Wasserstoff oder einem Wasserstoff enthaltenden Gas reduziert. Die Bedingungen bei der Aktivierung entsprechen im Allgemeinen den Reduktionsbedingungen, die bei der Reduktion angewandt werden. Durch die Aktivierung wird in der Regel die schützende Passivierungsschicht aufgehoben.

Die einzelnen Schritte und Methoden zur Herstellung von Aminierungskatalysatoren sind dem Fachmann bekannt und können den nachstehend genannten Offenlegungsschriften entnommen werden.

Bevorzugt werden folgende Katalysatoren in die MEG-Umsetzung eingesetzt:
Die in US 4,111,840 offenbarten Ni/Re-Katalysatoren.
Die in US 3,137,730 offenbarten Cu/Ni-Katalysatoren.
Die in DE 1 172 268 offenbarten Katalysatoren, die mindenstens eines der Aktivemtalle Cu, Fe, Ni und Co enthalten.
Die in WO 2007/093514 offenbarten geträgerten Ru/Co-Katalysatoren.
Die in WO 2013072289 offenbarten Katalysatoren, die neben Al, Cu, Ni und Co das Element Sn beinhalten.
Die in der WO 200908051, der WO 2009080508, der WO 200006749 und der WO 20008006750 offenbarten Katalysatoren, die neben Zr und Ni auch Cu, Sn, Co und/oder Fe beinhalten und als weitere Bestandteile Elemente wie V, Nb, S, O, La, B, W, Pb, Sb, Bi und In enthalten.
Die in den Anmeldungen mit der Anmeldenummer EP 1, EP 2 und EP3 offenbarten Katalysatoren.

Bevorzugt setzt man den Katalysator in Form kleiner Körper ein, wie Zylinder, Kugeln oder Tabletten. Bevorzugt weisen die Formkörper einen Durchmesser von gleich oder weniger als 10 mm auf, besonders bevorzugt von gleich oder weniger als 5 mm, ganz besonders bevorzugt von gleich oder weniger als 2 mm auf.

Molare Verhältnis der Edukte

Vorzugsweise enthält das eingesetzte Eduktgemisch einen molaren Überschuss an Ammoniak bezogen auf die eingesetzte MEG-Menge. Das molare Verhältnis NH3/MEG kann dabei 1 bis 100, bevorzugt 2 bis 50, besonders bevorzugt 7 bis 18 betragen.

### Reaktoren

Bevorzugte Reaktoren für die Umsetzung von MEG mit Ammoniak sind im Allgemeinen Rohrreaktoren. In den Rohrreaktoren ist der Aminierungskatalysator bevorzugt als Fließ- oder Festbett angeordnet.

Besonders bevorzugt erfolgt die Umsetzung von Ethylenglykol und/oder Monoethanolamin mit NH₃ in einem Rohrreaktor, in dem der Aminierungskatalysator als Festbett angeordnet ist. Alternativ erfolgt die Umsetzung vorteilhaft in einem Rohrbündelreaktor oder in einer Monostranganlage.Bei einer Monostranganlage kann der Rohrreaktor, in dem die Umsetzung erfolgt, aus einer Hintereinanderschaltung mehrerer (z.B. zweier oder dreier) einzelner Rohrreaktoren betehen. Optional ist hier vorteilhaft eine Zwischeneinspeisung von Feed (enthaltend das Edukt und/oder Ammoniak und/oder H₂) und/oder Kreisgas und/oder Reaktraustrag aus einem nachgeschalteten Reaktor möglich.

Es ist auch möglich, den Katalysator als Suspension zu halten, die in einen Rührkessel, Schlaufenreaktor oder einem Wirbelbett durch Bewegung der Flüssigkeit erzeugt wird.

### Temperatur und Druck

Bevorzugt erfolgt die Umsetzung von MEG bei Drücken von im Allgemeinen 5 bis 50 MPa (50-500 bar), bevorzugt 5 bis 40 MPa, besonders bevorzugt 20 bis 35 MPa, und Temperaturen von im Allgemeinen 50 bis 350 °C, besonders 100 bis 300 °C, bevorzugt 120 bis 270 °C, besonders bevorzugt 150 bis 220 °C, insbesondere 170 bis 200 °C.

Wenn der Reaktor adiabat betrieben wird, steigt im Allgemeinen die Temperatur in Abhängigkeit vom Umsatzes und des molaren Einsatzverhältnisses von MEG zu Ammoniak. So ist der adiabate Temperaturanstieg bei gleichem Umsatz niedriger, wenn die Ammoniakmenge höher ist. Der Temperaturanstieg in einem adiabaten Reaktor beträgt in der Regel 0 bis 200 °C, bevorzugt 5 bis 100 °C, besonders bevorzugt 7 bis 30 °C.

Der Wasserstoffpartialdruck beträgt vorzugsweise 0,25 bis 20 MPa (2,5 bis 200 bar), besonders bevorzugt 0,5 bis 15 MPa (5 bis 150 bar), ganz besonders bevorzugt 1 bis 10 MPa (10 bis 100 bar) und insbesondere bevorzugt 2 bis 5 MPa (20 bis 50 bar).

### Katalysatorbelastung

Die Katalysatorbelastung, ausgedrückt als an MEG eingesetzte Gewichtsmenge pro Zeiteinheit dividiert durch die eingesetzte Katalysatorgewichtsmenge, beträgt 0.05 bis 10 kg/kg h, bevorzugt 0.1 bis 5 kg/kg h, besonders bevorzugt 0.2 bis 1.5 kg/kg h.

### Austrag

Der Austrag aus der Stufe 1) enthält im Allgemeinen die Produkte der Aminierungsreaktion, nicht umgesetzte Edukte, wie MEG und Ammoniak, sowie Wasserstoff und Wasser.

Als Produkte der Aminierungsreaktion enthält der Austrag aus dem Aminierungsreaktor weiterhin die entsprechenden Ethanolamine und/oder Ethylenamine auf Basis von MEG.

Besonders bevorzugt enthält der Reaktionsaustrag nicht umgesetztes MEG, Ammoniak und Wasserstoff, sowie die Reaktionsprodukte MEA, EDA, PIP, AEEA, AEP, DETA und höhere Ethylenamine (unter höheren Ethylenamine werden Ethylenamine mit einem Siedepunkt größer oder gleich TETA bezeichnet), beispielsweise TETA und TEPA.
Weiterhin kann der Reaktionsaustrag NMEDA enthalten. NMEDA ist ein unerwünschtes Nebenprodukt. In vielen technischen Anwendungen wird eine Reinheit von EDA spezifiziert, bei der Anteil an NMEDA unter 500 Gew.-ppm liegt. In einer bevorzugten Ausführungsform wird deshalb in zwei weiteren Stufen, einer Stufe 4 (NMEDA-Abtrennung) und einer Stufe 5 (EDA-Reinigung) NMEDA abgetrennt (wie nachfolgend beschrieben).

### Ammoniakabtrennung - Stufe 2:

Die Gemische aus der MEG-Umsetzung enthalten in der Regel Ammoniak und Wasserstoff.

Die Menge an Ammoniak in den Reaktionsausträgen liegt typischerweise in dem Bereich von 50 bis 90 Gew.-%, besonders bevorzugt im Bereich von 60 bis 85 Gew.-% und ganz besonders bevorzugt im Bereich von 70 bis 80 Gew.-%.

Erfindungsgemäß wird in einer Stufe 2 Wasserstoff und Ammoniak aus dem Reaktionsaustrag aus Stufe 1) abgetrennt.

Erfindungsgemäß erfolgt die Abtrennung von Wasserstoff und/oder Ammoniak aus dem Reaktionsaustrag aus Stufe 1, dadurch, dass die Stufe 2 folgende Schritte umfasst:
2-1) Auftrennung des Reaktionsaustrags aus Stufe 1 in eine gasförmige Phase, welche Ammoniak und/oder Wasserstoff enthält, und eine flüssige Phase, welche Ethylenamine und/oder Alkanolamine enthält,
2-2) Überleitung der gasförmigen Phase aus Stufe 2-1) über ein oder mehrere Kondensatoren, wobei eine oder mehrere flüssige Phase erhalten werden, in denen Ammoniak angereichert ist, und eine gasförmige Phase erhalten wird, in der Wasserstoff angereichert ist,
2-3) Inkontaktbringen der gasförmigen Phase aus Stufe 2-2) mit MEG, so dass eine flüssige Phase, enthaltend MEG und Ammoniak, erhalten wird und eine gasförmige Phase, enthaltend Wasserstoff und optional Ammoniak, erhalten wird.

Überraschenderweise wurde gefunden, dass bei dem Inkontaktbringen der gasförmigen Phase aus Stufe 2-2), welche Wasserstoff und Ammoniak enthält, Wärme frei wird. Durch das erfindungsgemäße Verfahren kann MEG, welches in die Stufe 1) geführt werden soll, in der Stufe 2-3) vorgeheizt werden, wodurch Energie eingespart werden kann. Weiterhin erweist sich als vorteilhaft, dass sowohl die Waschflüssigkeit als auch das Ammoniak Edukte des erfindungsgemäßen Verfahrens sind, und somit zusammen ohne weitere Aufreinigung in das erfindungsgemäße Verfahren eingesetzt werden können.

Die Auftrennung des Reaktionsaustrags aus Stufe 1 erfolgt bevorzugt dadurch, dass man den Reaktionsaustrag aus Stufe 1) in einer Stufe 2-1) entspannt (Flash). Dazu wird der Reaktionsaustrag aus Stufe 1) bevorzugt in einen Entspannungsbehälter (Flash-Behälter) bzw. Abscheidebehälter geleitet.
Der Entspannungsbehälter wird vorzugsweise bei einem Druck im Bereich von 20 bis 80 bar, bevorzugt 30 bis 70 bar und insbesondere bevorzugt 40 bis 60 bar betrieben.
Die Entspannung auf den niedrigeren Druck erfolgt bevorzugt über ein Entspannungsventil und ggf. einen Einleitungsverteiler (Inlet Diffuser oder Inlet-Distributor).
Die sich bei der Entspannung bildene gasförmige Phase wird in der Regel im oberen Bereich des Entspannungsbehälters abgezogen. Vorzugsweise ist vor dem Dampfabzug eine Vorrichtung zum Abscheiden von Flüssigkeitstropfen, beispielsweise ein Maschengitter, angebracht. Die bei der Entspannung nicht verdampfende flüssige Phase wird in der Regel im unteren Bereich des Behälters gesammelt und abgezogen. Der Abzug kann dabei über ein Regelventil gesteuert werden, so dass im Entspannungsbehälter ein konstantes Flüssigkeitsniveau eingestellt werden kann.
Die flüssige Phase enthält im Allgemeinen die Komponenten des Reaktionsaustrags, die bei der Entspannung in der flüssigen Phase verbleiben. Dies sind insbesondere die in Stufe 1) gebildeten Ethylenamine und/oder Alkanolamine, sowie nicht umgesetztes MEG. Die flüssige Phase kann zudem Wasser enthalten.
Die sich bei der Entspannung bildene gasförmige Phase enthält im Allgermeinen Wasserstoff und/oder Ammoniak. Die Temperatur der gasförmigen Phase beträgt im Allgemeinen 50 bis 120°C, bevorzug 60 bis 100°C und besonders bevorzug 70 bis 90°C.

Die gasförmige Phase aus Stufe 2-1) wird in einer Stufe 2-2) über ein oder mehrere Kondensatoren geleitet. Vorzugsweise sind die ein oder mehrere Kondensatoren wassergekühlte Kondensatoren, insbesondere Rohrbündelkondensatoren oder Plattenwärmetauscher.
In den ein oder mehreren Kondensatoren wird die Temperatur der gasförmigen Phase aus Stufe 1) abgesenkt.

Durch die Absenkung der Temperatur wird in der Regel der Wasserstoff entsprechend der Taulinie des in den Kondensator eingeleiteten Gemisches in der Gasphase angereichert. Zudem wird im Kondensator eine flüssige Phase erhalten, in der Ammoniak angereichert ist.
Dadurch wird eine gasförmige Phase, welche Ammoniak und Wasserstoff enthält, und eine flüssige Phase, welche Ethylenamine und/oder Alkanolamine enthält, erhalten.
Die flüssigen Phasen, die an den ein oder mehreren Kondensatoren in Stufe 2-2) abgetrennt werden, werden bevorzugt getrennt oder zusammen in Stufe 1) zurückgeführt.
Als Kühlflüssigkeit für die ein oder mehreren Kondensatoren wird vorzugsweise Wasser verwendet. Das Kühlwasser weist bevorzugt eine Temperatur von 20 bis 90°C, bevorzugt 20 bis 50°C und insbesondere bevorzugt 20 bis 30°C auf.
Die ein oder mehrere Kondensatoren werden bevorzugt so ausgelegt, dass die Temperatur der gasförmigen Phase am Ausgang der ein oder mehreren Kondensatoren im Bereich von 25 bis 75°C, bevorzugt 30 bis 70°C und insbesondere bevorzugt 40 bis 60°C liegt.
In einer bevorzugten Ausführungsform besteht die Stufe 2-2) aus einem Kondensator.
In einer weiteren bevorzugten Ausführungsform besteht die Stufe 2-2) aus zwei Kondensatoren.

Die gasförmige Phase aus Stufe 2-2) wird in einer Stufe 2-3) mit MEG in Kontakt gebracht, so dass eine flüssige Phase erhalten wird, welche Ammoniak und MEG enthält, und eine gasförmige Phase erhalten wird, welche Wasserstoff und Ammoniak enthält.
Das Inkontaktbringen der gasförmigen Phase aus Stufe 2-2) mit MEG erfolgt im Allgemeinen in Apparaten, die für einen Stoffaustausch geeignet sind. Bevorzugt erfolgt das Inkontakbringen mit MEG in einer Waschkolonne und einem Venturiwäscher.
Bevorzugt erfolgt das Inkontaktbringen in einer Waschkolonne.
Bevorzugt enthält die Waschkolonne Einbauten zur Verbesserung des Stoffaustausches zwischen Flüssgkeit und Gas, insbesondere
Packungen, insbesondere strukturierte Packungen,
Böden, insbesondere Glockenböden, Zentrifugalböden oderSiebböden, oder
Füllkörper, insbesondere Raschigringe oder Pallringe.
Die Anzahl der theoretischen Trennstufe beträgt bevorzugt 5 bis 40, besonders bevorzugt 10 bis 30 und insbesondere bevorzugt 15 bis 25.
Die Temperatur des MEG, welches in dem Stoffaustauschapparat mit der gasförmigen Phase aus Stufe 2-2) in Kontakt gebracht wird, weist bevorzugt eine Temperatur im Bereich von 20 bis 80°C, besonders bevorzugt 30 bis 70°C und insbesondere bevorzugt 40 bis 60°C auf. In den bevorzugten Temperaturbereichen ist die Löslichkeit von Ammoniak in MEG ausreichend hoch. In dem Stoffaustauschapparat geht ein Teil der gasförmigen Komponenten der gasförmigen Phase aus Stufe 2-3) in die flüssige M EG-Phase über. Bevorzugt geht Ammoniak in die flüssige MEG-Phase über.
Bevorzugt wir ein M EG verwendet, welches einen Schwefelgehalt von weniger als 100 ppm enthält.. Besonders bevorzugt enthält das eingesetzte MEG weniger als 20 ppm Schwefel, ganz besonders bevorzugt weniger als 2 ppm Schwefel. Im Idealfall enthält das eingesetzte MEG gar keinen Schwefel. Es ist daher vorteilhaft, das frisch eingesetzte MEG vor Einführung in den Reaktionsteil so aufzuarbeiten, dass eventuell vorhandenes Schwefel abgetrennt wird. Dazu besteht die Möglichkeit, frisches MEG statt direkt in die Stufe 2-3), zunächst in Stufe b) oder Stufe 2 oder Stufe a), bevorzugt Stufe b), einzuleiten und nach der Aufrennung in Stufe b) in die Stufe 2-3) einzuleiten. Es wurde gefunden, dass durch die Rektifikation in Gegenwart von Aminen in Stufe b) nicht nur gegebenenfalls im Frisch-MEG vorhandene Hochsieder abgetrennt werden, sondern auch gegebenenfalls enthaltene Schwefelverbindungen, wie Sulfide, Sulfite, Mercaptane oder Thiole, die durch Reaktion oder Schleppwirkung mit den basischen Aminen Addukte (Hochsieder) bilden. Die voranstehend genannten bevorzugten Ausführungsformen ermöglichen somit auch den Einsatz eines MEG als Waschflüssigkeit, das keine sogenannte Faserqualität aufweist. In einer bevorzugten Ausführungsform wird MEG deshalb vor dem Einleiten in Stufe 2-3) bevorzugt in die Stufe b) eingeleitet und das in Stufe b) abgetrennte Gemisch D, welches das destillierte MEG enthält in Stufe 2-3) eingeleitet.
Die gasförmige Phase aus Stufe 2-3), welche nicht in die flüssige Phase übergegangen ist (Abgas), enthält vorzugsweise Wasserstoff und ggf. nicht in die MEG-Phase übergegangenes Ammoniak.

Bevorzugt wird die gasförmige Phase aus Stufe 2-3) in die Stufe 1) zurückgeführt.
Hierzu ist es im Allgemeinen erforderlich, dass die gasförmige Phase aus Stufe 2-3) auf den im Reaktor der Stufe 1) herrschenden Druck komprimiert wird.
Die Kompression erfolgt üblicherweise in einem Kompressor.
Es ist weiterhin bevorzugt einen Teil der gasförmigen Phase aus Stufe 2-3) vor der Kompression aus dem Verfahren auszuschleusen. Bevorzugt werden 10 bis 50 Gew.-%, besonders bevorzugt 15 bis 40 Gew.-% und insbesondere bevorzugt 20 bis 30 Gew.-% der gasförmigen Phase aus Stufe 2-3) aus dem Verfahren ausgeschleust.
Das Ausschleusen eines Teils der gasförmigen Phase hat den Vorteil, die Aufpegelung von Nebenprodukten, wie Methan und CO vermieden werden kann. Durch die Ausschleusung von CO kann die Standzeit der Aminierungskatalysatoren in Stufe 1) erhöht werden.
Die flüssige Phase aus Stufe 2-3) enthält MEG und Ammoniak.
Überraschenderweise wurde gefunden, dass sich die Temperatur des MEG beim Inkontaktbringen mit Ammoniak erhöht. Üblicherweise beträgt die Temperaturerhöhung des MEG in dem Stoffaustauschapparat 10 bis 100°C, bevorzugt 20 bis 80°C, besonders bevorzugt 30 bis 70°C und insbesondere bevorzugt 40 bis 60°C.
Es ist deshalb ganz besonders bevorzugt, das MEG, welches in Stufe 1) eingeleitet werden soll, vor Einleitung in die Stufe 1) in die Stufe 2-3) einzuleiten. Dadurch wird das MEG vorgewärmt, so dass weniger Energie aufgebracht werden muss, um MEG auf die in Stufe 1) herrschende Reaktionstemperatur zu bringen.

### Zusammensetzung des Austrags aus der Ammoniakabtrennung

Nach der Abtrennung von Ammoniak und ggf. Wasserstoff wird bevorzugt ein Gemisch erhalten, welches nicht umgesetztes MEG, sowie die Reaktionsprodukte MEA, EDA, PIP, AEEA, AEP, DETA und höhere Ethylenamine (unter höheren Ethylenamine werden Ethylenamine mit einem Siedepunkt größer oder gleich TETA bezeichnet), beispielsweise TETA und TEPA, enthält

Das nach der Ammoniakabtrennung erhaltene Gemisch, enthält bevorzugt 20 bis 75 Gew.-% EDA, besonders bevorzugt 30 bis 65 Gew.-% EDA und ganz besonders bevorzugt 35 bis 60 Gew.-% EDA.

Der Anteil an Ammoniak beträgt bevorzugt weniger als 5 Gew.-% Ammoniak, besonders bevorzugt weniger als 2 Gew.-% Ammoniak, besonders bevorzugt weniger als 1 Gew.-% Ammoniak und insbesondere bevorzugt weniger als 0,5 Gew.-%.

Der Anteil an höher siedenden Verbindungen mit einem Siedepunkt oberhalb des Siedepunkts von EDA, wie den Aminen MEA, DETA, AEP, AEEA, TETA, TEPA und höheren Ethylenaminen, sowie MEG, liegt bevorzugt im Bereich von 5 bis 90 Gew.-%, besonders bevorzugt im Bereich von 30 bis 85 Gew.-% und ganz besonders bevorzugt im Bereich von 40 bis 70 Gew.-%.

Das Gewichtsverhältnis von EDA zu Me-EDA beträgt vorzugsweise 1: 0,0005 (500 Gew.-ppm NMEDA) bis 1 : 0,2 (200 000 Gew.-ppm NMEDA), besonders bevorzugt 1 : 0,001 (1000 Gew.- ppm) bis 1 : 0,05 (50 000 Gew.-ppm NMEDA) und ganz besonders bevorzugt 1 : 0,005 (5000 Gew.-ppm NMEDA) bis 1 : 0,01 (10 000 Gew.-ppm NMEDA).

### MEA-Abtrennung - Stufe a):

Bevorzugt wird ein Gemisch enthaltend MEG, MEA, EDA und DETA sowie Leichtsieder, die einen Siedepunkt kleiner oder gleich PIP aufweisen, und Schwersieder, die einen Siedepunkt größer oder gleich AEEA aufweisen, in eine Auftrennungsstufe geleitet, in der die Aufrennung des eingeleiteten Gemisches in folgende Gemische erfolgt:
(i) ein Gemisch A, welches EDA und die Leichtsieder mit einem Siedepunkt kleiner oder gleich PIP, enthält; und
(ii) ein Gemisch B, welches MEA enthält; und
(iii) ein Gemisch C, welches MEG, DETA und die Schwersieder mit einem Siedepunkt größer oder gleich AEEA, enthält.

Die Auftrennung in Stufe a) kann in zwei hintereinander geschalteten Rektifikationskolonnen durchgeführt werden oder in einer einzigen Rektifikationskolonne.

### 2-Stufige MEA-Abtrennung in zwei Rektifikationskolonnen

Wird die Auftrennung in Stufe a) in zwei hintereinander geschalteten Rektifikationskolonnen a-1 und a-2 durchgeführt, so wird in der ersten Rektifikationskolonne a-1
(i) über Kopf oder einen Seitenabzug zwischen Einleitstelle und Kopf ein Gemisch A abgetrennt, welches EDA und die Leichtsieder mit einem Siedepunkt kleiner oder gleich PIP enthält, und
(ii) am Sumpf oder einen Seitenabzug zwischen Einleitstelle und Sumpf ein Gemisch BC abgezogen, welches MEA, MEG, DETA und die Schwersieder mit einem Siedepunkt größer oder gleich AEEA, enthält.

In der zweiten Rektifikationskolonne a-2 wird
(i) über Kopf oder einen Seitenabzug zwischen Einleitstelle und Kopf ein Gemisch B abgetrennt, welches MEA enthält, und
(ii) am Sumpf oder einen Seitenabzug zwischen Einleitstelle und Sumpf ein Gemisch C abgezogen, welches MEG, DETA und die Schwersieder mit einem Siedepunkt größer oder gleich AEEA enthält.

Verfahrensparameter der ersten Rektifikationskolonne a-1

Der Druck in der Rektifikationskolonne a-1 wird so gewählt, dass sich geeignete Sumpf- und Kopftemperaturen ergeben. Ein niedriger Druck erleichtert die Trennung durch Erhöhung der relativen Flüchtigkeit, wie dem Fachmann bekannt ist. Außerdem ergibt sich durch einen niedrigen Druck die Möglichkeit, Wärme bei einer niedrigen Temperatur am Verdampfer zu übertragen und dadurch Abfallwärme zu verwenden.

Die Sumpftemperatur beträgt Allgemeinen 100 bis 250 °C, bevorzugt 120 bis 200 °C, besonders bevorzugt 130 bis 180 °C.

Die Kopftemperatur beträgt im Allgemeinen 20 bis 200 °C, bevorzugt 40 bis 150 °C, besonders bevorzugt 50 bis 90 °C.

Die Anzahl der theoretischen Trennstufen beträgt im Allgemeinen 20 bis 100, bevorzugt 30 bis 80, besonders bevorzugt 35 bis 50.

Bevorzugt wird die Trennung in einer einzigen Rektifikationskolonne durchgeführt, wobei die leichtsiedende Fraktion über Kopf oder an einem oberen Seitenabzug, die mittelsiedende Fraktion an einem Seitenabzug zwischen Kopf und Sumpf und die hochsiedende Fraktion am Sumpf oder an einem unteren Seitenabzug abgeführt werden.

Als Einbauten können die üblichen, dem Fachmann bekannten Einbauten verwendet werden, wie beispielsweise Siebböden oder Glockenböden. Besonders bevorzugt ist die Anwendung von strukturierten Packungen, die den Betrieb bei einem besonders niedrigen Druckverlust und hoher Stufenzahl pro Meter Höhe erlauben.

Verfahrensparameter der zweiten Rektifikationskolonne a-2

Der Druck in der Rektifikationskolonne a-2 wird so gewählt, dass sich geeignete Sumpf- und Kopftemperaturen ergeben. Ein niedriger Druck erleichtert die Trennung durch Erhöhung der relativen Flüchtigkeit, wie dem Fachmann bekannt ist. Außerdem ergibt sich durch einen niedrigen Druck die Möglichkeit, Wärme bei einer niedrigen Temperatur am Verdampfer zu übertragen und dadurch Abfallwärme zu verwenden.

Die Sumpftemperatur beträgt Allgemeinen 100 bis 250 °C, bevorzugt 120 bis 200 °C, besonders bevorzugt 130 bis 180 °C.

Die Kopftemperatur beträgt im Allgemeinen 20 bis 200 °C, bevorzugt 40 bis 150 °C, besonders bevorzugt 50 bis 90 °C.

Die Anzahl der theoretischen Trennstufen beträgt im Allgemeinen 20 bis 100, bevorzugt 30 bis 80, besonders bevorzugt 35 bis 50.

### Einstufige MEA-Abtrennung in einer Rektifikationskolonne

Bevorzugt wird die Abtrennung in Stufe a) jedoch nur in einer einzigen Rektifikationskolonne a durchgeführt, wobei
(i) über Kopf oder an einem oberen Seitenabzug ein Gemisch A abgetrennt wird, welches EDA und die Leichtsieder mit einem Siedepunkt kleiner oder gleich PIP, enthält; und
(ii) an einem Seitenabzug zwischen Kopf und Sumpf ein Gemisch B abgetrennt wird, welches MEA enthält; und
(iii) am Sumpf oder an einem unteren Seitenabzug ein Gemisch C abgezogen wird, welches MEG, DETA und die Schwersieder mit einem Siedepunkt größer oder gleich AEEA, enthält.

Der Druck in der Rektifikationskolonne a wird so gewählt, dass sich geeignete Sumpf- und Kopftemperaturen ergeben. Ein niedriger Druck erleichtert die Trennung durch Erhöhung der relativen Flüchtigkeit, wie dem Fachmann bekannt ist. Außerdem ergibt sich durch einen niedrigen Druck die Möglichkeit, Wärme bei einer niedrigen Temperatur am Verdampfer zu übertragen und dadurch Abfallwärme zu verwenden.

Die Sumpftemperatur beträgt Allgemeinen 100 bis 250 °C, bevorzugt 120 bis 200 °C, besonders bevorzugt 130 bis 180 °C.

Die Kopftemperatur beträgt im Allgemeinen 20 bis 200 °C, bevorzugt 40 bis 150 °C, besonders bevorzugt 50 bis 90 °C.

Die Anzahl der theoretischen Trennstufen beträgt im Allgemeinen 20 bis 100, bevorzugt 30 bis 80, besonders bevorzugt 35 bis 50.

### Einstufige MEA-Abtrennung in einer Trennwandkolonne

In einer besonders bevorzugten Ausführungsform wird eine Rektifikationskolonne a-T mit Trennwand verwendet.

Bei einer Trennwandkolonne verläuft im Allgemeinen über einen Teil der Kolonnenhöhe eine vertikale Trennwand, welche den Querschnitt in zwei Abschnitte aufteilt. Oberhalb der Trennwand wird die Flüssigphase gesammelt und in einem wählbaren Verhältnis auf die beiden Kolonnenquerschnitte verteilt. Die Verwendung kann zu einer Verringerung der Investitionskosten und des Energiebedarfs bei der Auftrennung führen.
Vorzugsweise befindet sich die die Zulaufstelle auf der einen Seite der Trennwand und die Stelle vom mittleren Seitenabzug auf der anderen Seite der Trennwand.

Bevorzugt wird am Kopf der Trennwandkolonne ein Gemisch A abgetrennt, welches EDA und die Leichtsieder mit einem Siedepunkt kleiner oder gleich PIP, enthält.
Bevorzugt wird an einem Seitenabzug der Trennwandkolonne zwischen Kopf und Sumpf wird ein Gemisch B abgetrennt, welches MEA enthält.
Bevorzugt wird am Sumpf der Trennwandkolonne wird ein Gemisch C abgezogen, welches MEG, DETA und die Schwersieder mit einem Siedepunkt größer oder gleich AEEA, enthält.

Der Druck in der Trennwandkolonne a-T wird so gewählt, dass sich geeignete Sumpf- und Kopftemperaturen ergeben. Ein niedriger Druck erleichtert die Trennung durch Erhöhung der relativen Flüchtigkeit, wie dem Fachmann bekannt ist. Außerdem ergibt sich durch einen niedrigen Druck die Möglichkeit, Wärme bei einer niedrigen Temperatur am Verdampfer zu übertragen und dadurch Abfallwärme zu verwenden.

Die Sumpftemperatur beträgt Allgemeinen 100 bis 250 °C, bevorzugt 120 bis 200 °C, besonders bevorzugt 130 bis 180 °C.

Die Kopftemperatur beträgt im Allgemeinen 20 bis 200 °C, bevorzugt 40 bis 150 °C, besonders bevorzugt 50 bis 90 °C.

Die Anzahl der theoretischen Trennstufen beträgt im Allgemeinen 20 bis 100, bevorzugt 30 bis 80, besonders bevorzugt 35 bis 50.

### Weiterverarbeitung der Gemische aus der Stufe a)

Das Gemisch A, welches EDA und die Leichtsieder mit einem Siedepunkt kleiner oder gleich PIP, enthält wird in der Regel in zwei zusätzlichen Prozessstufen aufgearbeitet. In der sogenannten NMEDA-Abtrennung (Stufe 5) wird zunächst das unerwünschte Nebenprodukt NMEDA und Wasser abgetrennt. Die Stufe 5 kann wiederum in zwei Schritten durchgeführt werden, wobei in einem ersten Schritt die Abtrennung unter Bedingungen durchgeführt wird, unter denen EDA und Wasser ein hochsiedendes azeotropes Gemisch bilden, so dass NMEDA über Kopf abgetrennt werden kann. Der zweite Schritt wird vorzugsweise unter Bedingungen durchgeführt, bei denen Wasser und EDA kein Azeotrop bilden, so dass Wasser von EDA getrennt werden kann.

Das Gemisch aus der NMEDA-Abtrennung kann in einer weiteren Prozessstufe, der sogenannten EDA-Reinigung (Stufe 6) in seine Wertprodukte EDA und PIP getrennt werden.
Die NMEDA-Abtrennung (Stufe 5) und die EDA-Reinigung (Stufe 6) werden nachfolgend ausführlicher beschrieben.

Das Gemisch B, welches im Wesentlichen MEA enthält, kann in die MEG-Umsetzung (Stufe 1) zurückgeführt werden.

Bevorzugt wird das Gemisch B, welches im Wesentlichen MEA enthält, in eine separate Stufe 3 (MEA-Umsetzung) geleitet, in der MEA mit Ammoniak in Gegenwart von Wasserstoff und einem Aminierungskatalysator umgesetzt wird. Diese Ausführungsform hat den Vorteil, dass die Gesamtmenge AEEA, die im Verfahren gebildet wird, verringert werden kann, da MEA mit EDA in der MEG-Umsetzung zu AEEA reagieren kann.
Die Reaktionsbedingungen und Verfahrensparameter der MEA-Umsetzung entsprechen im Allgemeinen der zuvor beschriebenen MEG-Umsetzung (Stufe 1).
Im Allgemeinen wird bei der MEA-Umsetzung jedoch eine niedrigere Temperatur und ein niedrigerer Ammoniaküberschuss als bei der MEG-Umsetzung benötigt.
Wenn die MEA-Umsetzung in einer separaten Stufe 3 durchgeführt wird, so wird bevorzugt der Austrag aus der MEA-Umsetzung (Stufe 3) mit dem Austrag aus der MEG-Umsetzung (Stufe 1) zusammengeführt und gemeinsam der Ammoniakabtrennung (Stufe 2) zugeführt.

### MEG-Abtrennung - Stufe b):

Bevorzugt wird das Gemisch C, welches MEG, DETA und die Schwersieder mit einem Siedepunkt größer oder gleich AEEA enthält einer weiteren Auftrennungstufe b) zugeführt.

In einer bevorzugten Ausführungsform wird in die Stufe b) zusätzlich MEG eingeleitet, und zwar in der Menge, wie es für die Umsetzung in Stufe 1 benötigt wird. Dazu kann das MEG mit dem Gemisch C vor Einleitung in Stufe b) vermischt werden, oder das MEG und das Gemisch C können getrennt in Stufe b) eingeleitet werden und sich in Stufe b) vermischen. Diese Ausführungsform hat den Vorteil, dass das in Stufe 1 benötigte MEG in Stufe b) zusätzlich aufgereinigt werden kann. So kann in Stufe 1 ein MEG eingesetzt werden, dass einen niedrigen Schwefelgehalt aufweist, welches zu den voranstehend beschriebenen Vorteilen führen kann.

In Stufe b) wird das Gemisch C aus Stufe a) aufgetrennt in:
(i) ein Gemisch D, welches MEG enthält: und
(ii) ein Gemisch E, welches MEG, DETA und die Schwersieder mit einem Siedepunkt größer oder gleich AEEA, enthält.

Bevorzugt erfolgt die Auftrennung in einer Rektifikationskolonne b, wobei das Gemisch D bevorzugt als Kopfprodukt oder einem oberen Seitenabzug abgezogen wird und das Gemisch E bevorzugt als Sumpfprodukt oder einem unteren Seitenabzug abgezogen wird.

Der Druck am Kopf der Kolonne b beträgt im Allgemeinen 0.1 bis 3 bar, bevorzugt 0.2 bis 2 bar, besonders bevorzugt 0.25 bis 0.7 bar. Der Druck am Kopf der Kolonne b wird vorteilhaft so gewählt, dass die am Kondensator anfallende Kondensationswärme zum Betreiben eines Verdampfers an einem anderen Teil des Verfahrens eingesetzt werden kann, um die Gesamtenergiekosten zu verringern (Wärmeintegration).

Die Kopftemperatur der Rektifikationskolonne b beträgt im Allgemeinen 30 bis 220°C, bevorzugt 100 bis 200 °C, besonders bevorzugt 140 bis 190 °C.

Die Rektifikationskolonne b enthält im Allgemeinen 1 bis 20 theoretische Trennstufen, bevorzugt 2 bis 10 theoretische Trennstufen, besonders bevorzugt 3 bis 7 theoretische Trennstufen.

Die Trennung in Rektifikationskolonne b erfolgt in dem Fachmann bekannten Apparaturen, wie Glockenboden-, Siebodenkolonnen oder Kolonnen mit Füllkörpern oder strukturierter Packung. Bevorzugt werden druckverlustarme Einbauten wie strukturierte Packungen eingesetzt.

### Weiterverarbeitung der Gemische aus der Stufe b)

Das Gemisch D aus Stufe b) enthält im Wesentlichen MEG. Vorzugsweise weist das so erhaltene MEG einen niedrigen Schwefelgehalt auf. Bevorzugt enthält das Gemisch D, welches im Wesentlichen MEG enthält weniger als 100 ppm Schwefel in Form von Sulfid, Sulfit, oder einer organischen Schwefelverbindung wie Mercaptane oder Thiole. Besonders bevorzugt enthält das Gemisch D weniger als 20 ppm Schwefel, ganz besonders bevorzugt weniger als 2 ppm Schwefel. Die Einleitung eines solch schwefelarmen MEG in die MEG-Umsetzung (Stufe 1) hat die voranstehend erwähnten Vorteile.

Das Gemisch E aus Stufe b) enthält MEG, DETA und Schwersieder mit einem Siedepunkt größer oder gleich AEEA. Der Gehalt an DETA und MEG im Gemisch E entspricht dabei in etwa der Zusammensetzung des hochsiedenden Azeotropes DETA/MEG.

Ein besonderer Vorteil von Stufe b) ist, dass die anschließende Auftrennung des hochsiedenden Azeotropes DETA/MEG im Stufe d) erleichtert wird, da der in Stufe d) zugeführte Strom im Allgemeinen nur noch die Menge MEG enthält, wie sie der Zusammensetzung des Azeotrops DE-TA/MEG entspricht. Der Strom, der in Stufe d) eingeleitet wird, enthält somit in der Regel kein oder nur noch ein geringes Maß an überschüssigem MEG. Durch den geringeren Mengensrom, der in Stufe d) eingeleitet wird, verringert sich der Energiebedarf sowie die Apparategrößen. Weiterhin kann die thermische Belastung der Produkte in Schritt d) verringert werden, was zu einer höheren Qualität der Produkte führt.

### AEEA/Rückstand-Abtrennung - Stufe c):

Bevorzugt wird das Gemisch E aus Stufe b), welches MEG, DETA und Schwersieder mit einem Siedepunkt größer oder gleich AEEA enthält in einer Stufe c) aufgetrennt:
(i) in ein Gemisch F, welches MEG und DETA enthält; und
(ii) in ein Gemisch G, welches die Schwersieder mit einem Siedepunkt, größer oder gleich AEEA enthält.

Alternativ kann das Gemisch E aus Stufe b wie folgt aufgetrennt werden:
(i) in ein Gemisch F, welches MEG und DETA enthält; und
(ii) in ein Gemisch G1, welches AEEA enthält; und
(iii) in ein Gemisch G2, welches die Schwersieder mit einem Siedepunkt, größer oder gleich AEEA enthält.

Die Auftrennung in Stufe c) erfolgt vorzugsweise in einer Rektifikationskolonne c.

In der Rektifikationskolonne c wird bevorzugt das Gemisch F über Kopf oder einem oberen Seitenabzug abgezogen und das Gemisch G wird bevorzugt über Sumpf oder einem unteren Seitenabzug abgezogen.

Es ist auch möglich ein Gemisch F über Kopf abzuziehen und ein Gemisch G1, über einen mittleren Seitenabzug und ein Gemisch G2 über Sumpf oder einen unteren Seitenabzug.

Die Rektifikation in Stufe c wird im Allgemeinen in dem Fachmann bekannten Rektifikationseinrichtungen wie Glockenboden-, Siebbodenkolonnen oder Kolonnen mit Füllkörpern oder strukturierten Packungen durchgeführt.
Bevorzugt verwendet man strukturierte Packungen mit niedrigem Druckverlust.
Wird das Gemisch E in drei Fraktionen F, G1 und G2 getrennt, so wird als Rektifikationskolonne c bevorzugt eine Trennwandkolonne verwendet.

Die Anzahl der theoretischen Trennstufen beträgt 5 bis 50, bevorzugt 10 bis 30, besonders bevorzugt 15 bis 20.

Der Druck am Kopf der Kolonne beträgt 0.01 bis 3.0 bar, bevorzugt 0.05 bis 1 bar, besonders bevorzugt 0.1 bis 0.5 bar

Die Kopftemperatur der Rektifikationskolonne b beträgt im Allgemeinen 30 bis 220°C, bevorzugt 100 bis 200 °C, besonders bevorzugt 120 bis 180 °C.

### Weiterverarbeitung der Gemische aus der Stufe c)

Das Gemisch G, welches die Schwersieder mit einem Siedepunkt, größer oder gleich AEEA enthält wird im Allgemeinen in einer weiteren Auftrennungsstufe, wie beispielsweise einer Rektifikation, einer Destillation oder einer einfachen Verdampfung an einem Umlauf-, Fallfilm-, oder Dünnschichtverdampfer aufgearbeitet, um AEEA von Hochsiedern mit einem Siedepunkt größer als AEEA zu trennen.

Das Gemisch G1, welches im Wesentlichen AEEA enthält, kann auch direkt einer weiteren Verwendung zugeführt werden. Beispielsweise kann AEEA als Syntheschemikalie zur Herstellung anderer chemischer Verbindungen, wie Kraftstoff- und Öladditive, Chelatliganden, Tenside, Coatings, Weichspüler, Urethane oder andere verwendet werden. Es ist auch möglich, dass AEEA weiter aufzureinigen, wenn eine besonders hohe Qualität gefordert wird.

Das Gemisch G2, welches Hochsieder mit einem Siedepunkt größer als AEEA enthält, kann ebenfalls einer Verwendung zugeführt werden, beispielsweise als Asphaltadditiv, Korrosionsinhibitor, Kraftstoff- und Öladditiv, Tenside oder als Härter für Epoxysysteme.

### DETA-Abtrennung - Stufe d):

Bevorzugt wird das Gemisch F aus Stufe c) durch Extraktivdestillation mit Triethylenglykol (TEG) aufgetrennt in:
(i) ein Gemisch H, welches MEG enthält; und
(ii) ein Gemisch I, welches DETA und TEG enthält.

Vor der Einleitung des Gemischs F in Stufe d) wird dem Gemisch F TEG zugeführt.

Die Extraktivdestillation mit TEG als selektivem Lösungsmittel für DETA wird bevorzugt in der Weise betrieben, dass das molare Verhältnis von TEG zu DETA im Gemisch F nach Zuführung des TEG im Bereich von 1:1 bis 10:1 liegt, besonders bevorzugt 2:1 bis 8:1 und ganz besonders bevorzugt 3:1 bis 5:1 liegt.

Die Extraktivedestillation in Stufe d) wird bevorzugt in einer Rektifikationskolonne d durchgeführt

Bevorzugt wird am Kopf oder einem oberen Seitenabzug das Gemisch H abgezogen, während Gemisch I als Sumpfprodukt oder aus einem unteren Seitenabzug abgezogen wird.

Die Rektifikation in Stufe d) wird bevorzugt in dem Fachmann bekannten Rektifikationseinrichtungen wie Glockenboden-, Siebbodenkolonnen oder Kolonnen mit Füllkörpern oder strukturierten Packungen durchgeführt. Bevorzugt verwendet man strukturierte Packungen mit niedrigem Druckverlust.

Die Anzahl der theoretischen Trennstufen beträgt im Allgemeinen 10 bis 100, bevorzugt 20 bis 60, besonders bevorzugt 30 bis 50.

Der Druck am Kopf der Kolonne beträgt im Allgemeinen 0.005 bis 1.0 bar, bevorzugt 0.01 bis 0.2 bar, besonders bevorzugt 0.02 bis 0.1 bar

Die Kopftemperatur der Rektifikationskolonne d beträgt im Allgemeinen 50 bis 220°C, bevorzugt 70 bis 160 °C, besonders bevorzugt 80 bis 130 °C.

### Weiterverarbeitung der Gemische aus der Stufe d)

Das Gemisch H, welches im Wesentlichen MEG enthält, wird vorzugsweise in die MEG-Umsetzung zurückgeführt.

Das voranstehend beschriebene Verfahren kann optional mit weiteren Stufen zu einem besonders vorteilhaften Gesamtverfahren verbunden werden.

So wird vorzugsweise das in Stufe d) erhaltene Gemisch I, welches DETA und TEG enthält, in eine weitere Stufe 4 (TEG-Abtrennung) eingeleitet in der eine Auftrennung erfolgt in:
(i) ein Gemisch J, welches DETA enthält; und
(ii) ein Gemisch K, welches TEG enthält.

Das Gemisch A aus Stufe a) wird vorzugsweise in einer Stufe 5 (EDA-Entwässerung) eingeleitet, in der eine Auftrennung in:
(i) ein Gemisch L, welches NMEDA und Wasser enthält; und
(ii) ein Gemisch M, welches Wasser enthält; und
(iii) ein Gemisch N, welches EDA und PIP enthält.

Das Gemisch N aus Stufe 5 wird vorzugsweise in eine weiteren Stufe 6 (EDA-Reinigung) in folgende Gemische aufgetrennt:
(i) ein Gemisch O, welches EDA enthält; und
(ii) ein Gemisch P, welches PIP enthält; und
(iii) ein Gemisch Q, welches einen Rückstand enthält.

### Optionale Stufen:

### TEG-Abtrennung - Stufe 4

Vorzugsweise wird das in Stufe d) erhaltene Gemisch I, welches DETA und TEG enthält, in eine weitere Stufe 4 eingeleitet in der eine Auftrennung erfolgt in:
(i) ein Gemisch J, welches DETA enthält; und
(ii) ein Gemisch K, welches TEG enthält.

Die Stufe 4 wird bevorzugt in einer Rektifikationskolonne durchgeführt, bei der vom Kopf oder aus einem oberen Seitenabzug das Gemisch J abgezogen wird und das Gemisch K als Sumpfprodukt oder aus einem unteren Seitenabzug abgezogen wird.

Die Rektifikation in Stufe 4 wird bevorzugt in dem Fachmann bekannten Rektifikationseinrichtungen wie Glockenboden-, Siebbodenkolonnen oder Kolonnen mit Füllkörpern oder strukturierten Packungen durchgeführt. Bevorzugt verwendet man strukturierte Packungen mit niedrigem Druckverlust.

Die Anzahl der theoretischen Trennstufen in Kolonne e beträgt im Allgemeinen 5 bis 60, bevorzugt 10 bis 50, besonders bevorzugt 20 bis 40.

Der Druck am Kopf der Kolonne e beträgt im Allgemeinen 0.005 bis 1.0 bar, bevorzugt 0.01 bis 0.2 bar, besonders bevorzugt 0.02 bis 0.1 bar

Die Kopftemperatur der Rektifikationskolonne e beträgt im Allgemeinen 50 bis 220°C, bevorzugt 70 bis 160 °C, besonders bevorzugt 80 bis 130 °C.

### Weiterverarbeitung der Gemische aus der Stufe 4

Das Gemisch J, welches im Wesentlichen DETA enthält, kann einer weiteren Verwendung zugeführt werden, beispielsweise als Chelatligand, als Härter für Epoxidsysteme und als Zwischenprodukt zur Herstellung von Pflanzenschutzmitteln und Pharmazeutika.

Das Gemisch K, welches im Wesentlichen TEG enthält, wird bevorzugt, wie voranstehend aufgeführt, mit dem Gemish F aus Stufe c) vor Einleitung in die Stufe d) vermischt.

### EDA-Entwässerung - Stufe 5

Das Gemisch A aus Stufe a) wird vorzugsweise in eine Stufe 5 (EDA-Entwässerung) eingeleitet, in der eine Auftrennung in:
(iv) ein Gemisch L, welches NMEDA und Wasser enthält; und
(v) ein Gemisch M, welches Wasser enthält; und
(vi) ein Gemisch N, welches EDA und PIP enthält.

Die Trennung wird bevorzugt in einer Sequenz von zwei Rektifikationskolonnen 5-1 und 5-2 durchgeführt.

### NMEDA-Abtrennungskolonne 5-1

In der Rektifikationskolonne 5-1 wird bevorzugt das Gemisch L über Kopf abgetrennt wird und über Sumpf ein Gemisch MN, welches den wesentlichen Anteil an EDA als hochsiedendes Azeotrop mit Wasser und Piperazin enthält.
Die Sumpftemperatur in 5-1 beträgt bevorzugt weniger als 170 °C, besonders bevorzugt weniger als 160 °C, ganz besonders bevorzugt weniger als 155 °C beträgt.
Die Rektifikationstemperatur wird in der Regel durch Einstellung eines geeigneten Drucks bei der Rektifikation erreicht.

Vorzugsweise wird die Destillation unter Bedingungen durchgeführt, in denen Wasser und EDA ein hochsiedendes Azeotrop bilden. Dazu wird dem Gemisch ggf. zusätzliches Wasser zugeführt, wie sie für die Bildung eines hochsiedenden Azeotrops erforderlich ist. Bei Vorhandensein anderer Stoffe, die ein hochsiedendes Azeotrop mit Wasser bilden, muss zusätzlich mindestens die Wassermenge vorhanden sein, die der jeweiligen Konzentration der jeweiligen Komponente, die ein hochsiedendes Azeotrop mit Wasser bildet, entspricht.
Die Bestimmung der azeotropen Zusammensetzungen ist dem Fachmann geläufig. Für weitere Einzelheiten wird auf die EP 2 507 202 verwiesen.

Vorzugsweise wählt man einen möglichst niedrigen Druck für die Rektifikation, und zwar besonders vorzugsweise einen solchen, bei dem eine Kondensation des am Kopf anfallenden Dampfgemisches unter technisch üblichen Bedingungen erfolgt, d.h. einer Temperatur bei der noch mit Kühlwasser oder durch Umgebungsluftkühlung kondensiert werden kann. Dies sind üblicherweise Kopftemperaturen von 20°C und mehr, bevorzugt 30°C und mehr und besonders bevorzugt 35°C und mehr. Bevorzugt erfolgt die Kondensation in einem Temperaturbereich von 20 bis 60 °C, bevorzugt 30 bis 55 °C, besonders bevorzugt 40 bis 50 °C.

Bevorzugt wird am Kopf der Kolonne ein Druck von 2,5 bar und weniger, bevorzugt 1,6 bar und weniger und ganz besonders bevorzugt 1 bar und weniger eingestellt.
Enthält der Eintrag in die Rektifikationskolonne höher siedende Amine, so ist es im Allgemeinen erforderlich den Kopfdruck abzusenken, um die bevorzugte Temperatur im Sumpf der Kolonne zu erreichen.

Die Rektifikation kann in dem Fachmann bekannten Apparaturen, wie Glockenboden- , Siebbodenkolonnen oder Kolonnen mit Füllkörpern oder strukturierten Packungen erfolgen. Bevorzugt werden druckverlustarme Einbauten, wie strukturierte Packungen eingesetzt, beispielsweise als Blechpackung wie Mellapak 250 Y oder Montz Pak (Typ B1-250). Es kann auch eine Packung mit geringerer oder erhöhter spezifischer Oberfläche vorliegen, oder es kann eine Gewebepackung oder eine Packung mit anderer Geometrie, wie Mellapak 252.Y, verwendet werden. Vorteilhaft bei der Verwendung dieser destillativen Einbauten sind der geringe Druckverlust und der geringe spezifische Flüssig-Hold-up im Vergleich zu beispielsweise Ventilböden. Die Einbauten können in ein oder mehreren Betten vorliegen.

Die Rektifikationskolonne enthält bevorzugt 35 bis 140 theoretische Trennstufen, besonders bevorzugt 50 bis 120 theoretische Trennstufen und ganz besonders bevorzugt 60 bis 100 theoretische Trennstufen.

Der Eintrag in die Rektifikationskolonne wird vorzugsweise in einem räumlichen Bereich zwischen 25% und 95%der theoretischen Böden der Rektifikationskolonne zugeführt (von unten gezählt), besonders bevorzugt in einem räumlichen Bereich zwischen 60% und 90% der theoretischen Böden der Rektifikationskolonne. Beispielsweise kann die Zuführung-oberhalb der Mitte der theoretischen Böden erfolgen

Zur Verbesserung der Abtrennung von NMEDA wird vorzugsweise das am Kondensator anfallende Kondensat zu mehr als 30 %, bevorzugt zu mehr als 50%, in den Kopf der Rektifikationskolonne zurückgeführt. Der Rest wird aus dem Verfahren ausgeschleust und in der Regel einem Sammelbehälter und von dort in im Allgemeinen einer Entsorgung, vorzugsweise einer Kläranlage, zugeführt

Das Gemisch L, welches im Wesentlichen Wasser und NMEDA und ggf. Spuren von EDA enthält, wird vorzugsweise im oberen Bereich der Kolonne, besonders bevorzugt am Kopf der Kolonne entnommen und einem Kondensator zugeführt. Als Kondensator können beispielsweise Kondensatoren mit Kühlschlange oder Wendelrohr, Doppelrohrkühler sowie Rohrbündelwärmetauscher eingesetzt werden.

Zur Verbesserung der Abtrennung von NM EDA wird vorzugsweise das am Kondensator anfallende Kondensat zu mehr als 30 %, bevorzugt zu mehr als 50%, in den Kopf der Rektifikationskolonne zurückgeführt. Der Rest wird aus dem Verfahren ausgeschleust und in der Regel einem Sammelbehälter und von dort in im Allgemeinen einer Entsorgung, vorzugsweise einer Kläranlage, zugeführt.

Im unteren Bereich der Kolonne, vorzugsweise aus dem Sumpf oder einen unteren Seitenabzug, wird ein Gemisch MN abgezogen, welches den wesentlichen Anteil an EDA als hochsiedendes Azeotrop mit Wasser und Piperazin enthält.

### EDA-Entwässerungskolonne 5-2

Das Gemisch MN wird vorzugsweise in eine weitere Rektifikationskolonne 5-2 geleitet, in der vorzugsweise über Kopf oder einen oberen Seitenabzug das Gemisch M abgezogen wird und am Sumpf oder einen unteren Seitenabzug das Gemisch N abgezogen wird.

Vorzugsweise wird die EDA-Entwässerungskolonne 5-2 unter Bedingungen betrieben, bei denen EDA und Wasser ein zeotropes Gemisch bilden.

Der Druck in Kolonne 5-2 wird im Allgemeinen so eingestellt, dass die Sumpftemperatur höher als180 °C, bevorzugt höher als 190 °C, besonders bevorzugt höher als 200 °C beträgt.

In der bevorzugten Ausführungsform liegt deshalb der absolute Druck am Kopf der Rektifikationskolonne bevorzugt im Bereich von 4 bis 30 bar, besonders bevorzugt 6 bis 10 bar und insbesondere bevorzugt 7 bis 9 bar.

Der Zulauf erfolgt besonders bevorzugt in einem räumlichen Bereich zwischen 50% und 100% der theoretischen Böden der Rektifikationskolonne. Beispielsweise kann die Zuführung auf den Kopf der Kolonne erfolgen. Die optimale Zulaufstelle kann vom Fachmann mit den üblichen Berechnungswerkzeugen ermittelt werden.

Die Anzahl der theoretischen Trennstufen liegt im Allgemeinen im Bereich von 10 bis 80, vorzugsweise 30 bis 60.

In einer bevorzugten Ausführungsform weist die EDA-Entwässerungskolonne einen Kondensator auf, der in der Regel bei einer Temperatur betrieben, in dem der überwiegende Teil des Wassers bei dem entsprechenden Kopfdruck kondensiert wird.
In der Regel liegt die Betriebstemperatur des Kondensators im Bereich von 150 bis 230°C, vorzugsweise 160 bis 195°C.

Im Kondensator fällt in der Regel ein Kondensat an, welches überwiegend Wasser enthält. Vorzugsweise wird das am Kondensator anfallende Kondensat zu mehr als 50 %, bevorzugt zu mehr als 65 %, in den Kopf der Rektifikationskolonne zurückgeführt.

Das nicht zurückgeführte Kondensat kann im Allgemeinen direkt der Entsorgung zugeführt werden, beispielsweise durch Einleiten in eine Abwasseraufbereitungsanlage.

In einer weiteren bevorzugten Ausführungsform wird das nicht in die EDA-Entwässerung zurückgeführte Kondensat in den Sumpf der NMEDA-Abtrennungskolonne 5-1 eingeleitet. Dies hat den Vorteil, dass die Wassermenge in der NMEDA-Abtrennungskolonne erhöht wird, so dass die NMEDA-Abtrennungskolonne so viel Wasser enthält, wie es für die Bildung eines hochsiendenen Azeotrops von EDA und Wasser erforderlich ist.

In einer bevorzugten Ausführungsform wird das am Kopf der EDA-Entwässerungskolonne 5-2 abgezogene Gemisch M, welches im Wesentlichen Wasser enthält, nicht kondensiert und in Form von Brüden (unter "Brüden" versteht man hier den am Kopf einer Kolonne anfallenden, in der Regel dampfförmigen Strom, bevor er einem Kondensator zugeführt wird) in die NMEDA-Abtrennungskolonne 5-1 eingeleitet.

Die Einleitung der Brüden kann direkt in den Abtriebsteil der Kolonne 5-1, vorzugsweise den Sumpf erfolgen. Hierbei ist es vorteilhaft, den Brüdenstrom von 5-2 zu 5-1 mit Hilfe eines Regelventils oder einer Regelklappe auf den niedrigeren Druck zu drosseln oder die Drosselung anhand einer Turbine vorzunehmen, die einen Motor antreibt, der Strom erzeugt.
Die Brüden können als Heizdampf in einen Verdampfer der Kolonne 5-1 geleitet werden.

In beiden Fällen wird der Energiebedarf am Verdampfer der Kolonne 5-1 um einen erheblichen Betrag vermindert.

### EDA-Reinigung - Stufe 6

Das Gemisch N aus Stufe 5, welches EDA und PIP enthält, wird bevorzugt in einer weiteren Stufe 5 wie folgt aufgetrennt:
(iii) ein Gemisch O, welches EDA enthält; und
(iv) ein Gemisch P, welches PIP enthält; und
(v) ein Gemisch Q, welches einen Rückstand enthält.

Die Auftrennung in Stufe 6 wird vorzugsweise in einer Rektifikationskolonne 6-1 durchgeführt.

Hierbei wird bevorzugt über Kopf oder einen oberen Seitenabzug oberhalb vom Zulauf ein Gemisch O abgezogen, welches im Wesentlichen EDA enthält.

Am Seitenabzug oder über den Sumpf wird bevorzugt ein Gemisch P abgezogen, welches im Wesentlichen PIP enthält.

Aus dem Sumpf kann optional ein Gemisch Q als Rückstand abgezogen werden, welches im Allgemeinen Schwersieder mit einem höheren Siedepunkt als PIP enthält.

Die Trennung erfolgt in dem Fachmann bekannten Kolonnen, wie Glockenboden-, Siebbodenkolonnen oder Kolonnen mit Füllkörpern oder strukturierten Packungen.

Die Rektifikationskolonne enthält 10 bis 70 theoretische Trennstufen, bevorzugt 20 bis 60 theoretische Trennstufen, besonders bevorzugt 30 bis 50 theoretische Trennstufen.

Der Druck am Kopf der Kolonne beträgt 0.1 bis 10 bar, bevorzugt 0.5 bis 5 bar, besonders bevorzugt 1.0 bis 3 bar.

Es ist möglich, den Druck der Kolonne und somit die Kopftemperatur am Kondensator so zu wählen, dass die anfallende Kondensationswärme zur Beheizung eines weiteren Verdampfers im Verfahren verwendet werden kann, um Energiekosten einzusparen.

### Vorteile

Durch das erfindungsgemäße Verfahren können Ressourcen, wie Energie und Einsatzstoffe, insbesondere Verbrauchsmittel, wie Waschflüssigkeit, eingespart werden. Überraschenderweise wurde gefunden, dass bei der Verwendung von MEG als Waschlösung zur Abtrennung von Ammoniak aus der gasförmigen Phase aus Stufe 2-2), welche Wasserstoff und Ammoniak enthält, Wärme frei wird. Durch das erfindungsgemäße Verfahren kann MEG, welches in die Stufe 1) geführt werden soll, in der Stufe 2-3) vorgeheizt werden, wodurch Energie eingespart werden kann. Weiterhin erweist sich als vorteilhaft, dass sowohl die Waschflüssigkeit als auch das Ammoniak Edukte des erfindungsgemäßen Verfahrens sind, und somit zusammen ohne weitere Aufreinigung in das erfindungsgemäße Verfahren eingesetzt werden können.

Das erfindungsgemäße Verfahren wird anhand von nachfolgenden Beispielen erläutert.

### Beispiel 1:

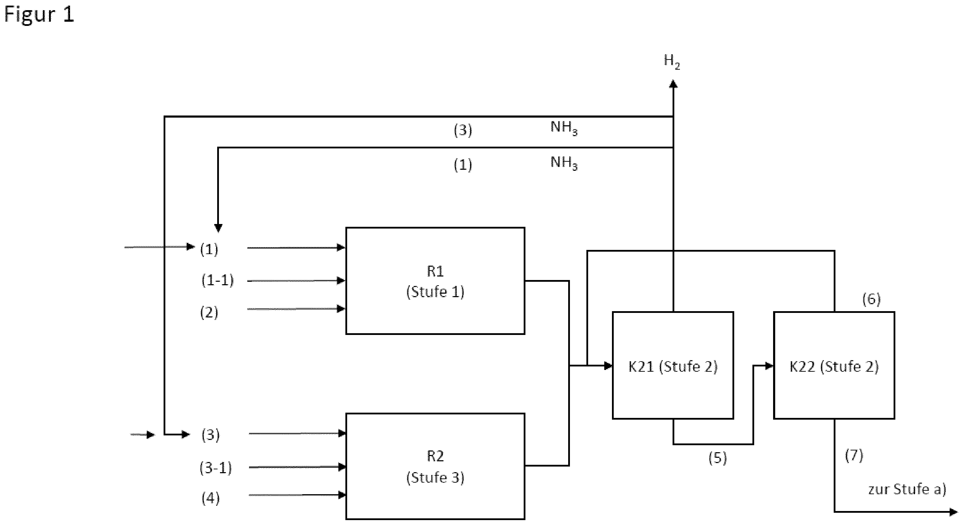

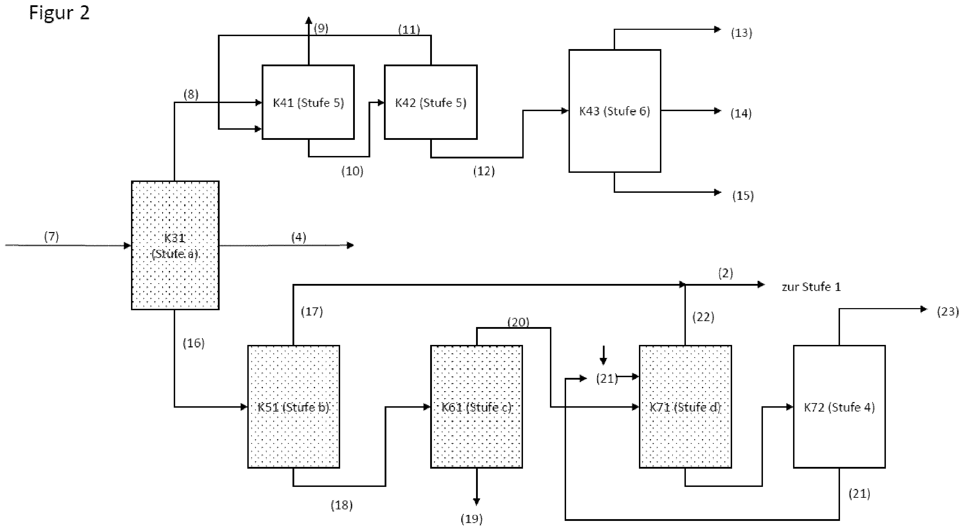

Beispiel 1 wurde gemäß der in Figur 1 und Figur 2 aufgezeigten Anordnung durchgeführt.

Die Herstellung des Gemisches enthaltend MEG, MEA, EDA und DETA erfolgte in zwei parallelen Stufen (Stufe 1: MEG-Umsetzung und Stufe 3; MEA-Umsetzung), wie in Figur 1 dargestellt.

In Stufe 1 wird ein Strom (1) mit 53.5 kg/h Ammoniak in flüssiger Forme bei 190 bar mit einem Strom (2) bestehend aus 13.9 kg/h MEG bei 190 bar und einem weiteren Strom (1-1) bestehend aus 350 g/h Wasserstoff gemischt, auf 175 °C erhitzt und durch einen Rohrreaktor (R1) enthaltend 20 kg eines Ni-, Co-, Cu-, Ru- und Sn-haltigen Katalysators.
Die Katalysatorherstellung erfolgte dabei wie folgt: Ein Katalysatorvorläufer wurde gemäß Beispiel B3 der WO 2013/072289 hergestellt. Die so erhaltenen Tabletten (3*3 mm) wurden zu 1-2 mm Splitt zerkleinert. Die maximale Wasseraufnahmekapazität des Splitts betrug 0,30 mL/g. Es wurde eine Metallsalzlösung hergestellt. Hierzu wurden 20,25 g Cobaltnitrathexahydrat (20,25 Gew.-% Co) in heißem Wasser gelöst und 37,91 g Ru-nitrosylnitratlösung zugegeben. Die so erhaltene Lösung wurde mit VE-Wasser auf 71 ml aufgefüllt und in ein Sprühgefäß überführt. Der Splitt wurde in einer Tränkapparatur mit einer Menge besprüht, die 95% der maximalen Wasseraufnahme des Splitts entspricht. Um die homogene Aufnahme der Tränklösung zu gewährleisten, wurde der Splitt noch weitere 30 min nachrotiert. Im Anschluss wurde der Katalysatorsplitt für 16 h bei 120°C im Umlufttrockenschrank getrocknet. Im Anschluss an die Trocknung wurde der Katalysatorvorläufer bei maximal 200°C in einem Strom aus Stickstoff und Wasserstoff reduktiv calciniert.

In Stufe 3 wurde ein Strom (3) mit 4.7 kg/h Ammoniak in flüssiger Form bei 190 bar mit einem Strom (4) bestehend aus 1.88 kg/h MEOA und einem weiteren Strom (3-1) bestehend aus 65 g/h Wasserstoff bei 190 bar gemischt, auf 150 °C erhitzt und durch einen Reaktor (R2) enthaltend 5 kg Katalysator (Herstellung: s.o) geleitet.

Die Reaktionsausträge aus Stufe 1 und Stufe 3 wurden zusammengeführt und in die Ammoniakabtrennung (Stufe 2) geleitet.

Die Stufe 2 wurde in zwei Schritten in zwei Rektifikationskolonnen (K21 und K22) durchgeführt. In einem ersten Schritt wurde die vereinigten Gemische aus Stufe 1 und 3 in eine Rektifikationskolonne (K21) mit nur einem Abtriebsteil mit 2 theoretischen Trennstufen geleitet. K21 wurde bei einem Kopfdruck von 20 bar und einer Sumpftemperatur von 165 C betrieben. Am Kopf der K21 wurde eine Temperatur von 49 °C gemessen. Über Kopf von K21 wurde der enthaltene Ammoniak abgetrennt und als Ströme (1) und (3) in die Stufen 1 und 3 zurückgeführt, wobei ein kleiner Strom frischen Ammoniaks zur Ergänzung von Verlusten hinzugeführt wurde. Am Kondensator der K21 entstand zudem eine kleine Menge Abgas, bestehend hauptsächlich aus der in den Ammoniakzuläufen, Ströme (1) und (3) enthaltenen Wasserstoffmenge. Der Sumpfaustrag von Kolonne K21, Strom (5), wurde in einer weiteren Kolonne (K22) eingeleitet, die nur einen Abtriebsteil mit 5 theoretischen Trennstufen enthielt. K21 wurde bei einem Kopfdruck von 4 bar und einer Sumpftemperatur von 173 °C betrieben. Am Kopf der K21 wurde ein Gemisch aus Ammoniak und Wasser bei 55 °C als Strom (6) abgezogen und in die Kolonne K21 zurückgeführt. Der ammoniakfreie Sumpfaustrag der Kolonne K22 wurde als Strom (7) wurde in das erfindungsgemäße Verfahren eingeleitet.

### Stufe a)

Der Austrag aus Stufe 2 (Strom (7)) wurde in die Stufe a) geleitet, wie in Figur 2 dargestellt. Die Stufe a) ist als Trennwandkolonne ausgestaltet, die die bei einem Kopfdruck von 150 mbar betrieben wurde. Kolonne K31 enthielt eine geordnete Packung entsprechend 16 theoretischen Trennstufen als Abtriebsteil unterhalb des Trennbleches, 5 theoretischen Trennstufen auf der Zulaufseite des Trennbleches unterhalb der Zulaufstelle, 10 theoretischen Trennstufen auf der Zulaufseite des Trennbleches oberhalb der Zulaufstelle, 12 theoretischen Trennstufen auf der Abzugsseite des Trennbleches unterhalb der Seitenabzugsstelle, 8 theoretischen Trennstufen auf der Abzugsseite des Trennbleches oberhalb der Seitenabzugsstelle und 3 theoretischen Trennstufen als Verstärkungsteil oberhalb des Trennbleches. Die Kopftemperatur an K31 betrug 73 °C, die Sumpftemperatur 146 °C und die Rücklaufmenge 10.6 kg/h. Am Seitenabzug der K31 wurde ein Strom enthaltend 1.88 kg/h MEA abgezogen (Gemisch B), der als Strom (4) auf in die Stufe 3 geleitet wurde.

### Stufe 5) NMEDA-Abtrennung

Am Kopf der Kolonne K31 wurde ein Strom (8) enthaltend EDA und Leichtsieder mit einem Siedepunkt kleiner oder gleich PIP (Gemisch A), abgezogen, der in die NMEDA-Abtrennung (Stufe 5) geleitet wurde, wie in Figur 2 dargestellt.

Die Stufe 5 wurde in zwei Schritten in zwei Rektifikationskolonnen durchgeführt.

Die erste Rektifikationskolonne K41 wurde bei einem Kopfdruck von 150 mbar betrieben. Kolonne K41 enthielt eine geordnete Packung entsprechend 13 theoretischen Trennstufen im Verstärkungs- und 67 theoretischen Trennstufen im Abtriebsteil. Die Kopftemperatur betrug 55 °C, die Sumpftemperatur 79 °C und die Rücklaufmenge 5 kg/h. Über Kopf wurden 3.3 kg/h Wasser als Strom (9) abgezogen, die 100 ppm EDA und 1217 ppm NMEDA (Gemisch L) enthielten. Über Sumpf der K41 wurde ein Strom (10) mit 12 kg/h, enthaltend 1 ppm NMEDA abgezogen. Strom (10) wurde in eine zweite Rektifikationskolonne K42 geleitet, die aus einem Abtreibsteil mit geordneter Packung entsprechend 60 theoretischen Trennstufen bestand. Der Kopfdruck betrug 8.5 bar, die Kopftemperatur 189 °C und die Sumpftemperatur 204 °C. Die K42 enthielt keinen Kondensator, die gesamten Brüden der Kolonne wurden als Strom (11) in den Sumpf der K41 eingeleitet. Über Kopf der K42 wurde ein Strom (6) enthaltend Wasser (Gemisch M) abgezogen. Über Sumpf der K42 wurden 4.6 kg/h als Strom (12) enthaltend EDA, PIP, sowie 0.35 % Wasser abgezogen und in die EDA-Abtrennung (Stufe 6) geleitet.

### Stufe 6) EDA-Abtrennung

Die Stufe 6 wurde in einem Schritt in einer Rektifikationskolonne durchgeführt, wie in Figur 2 dargestellt.

Das Gemisch aus Stufe 5 wurde in eine Rektifikationskolonne K43 eingeleitet, die bei einem Kopfdruck von 1050 mbar betrieben wurde und einen unteren, dampfförmigen Seitenabzug aber kein Trennblech enthielt. Kolonne K43 enthielt geordnete Packung entsprechend 25 Trennstufen im Verstärkungsteil, 15 theoretische Trennstufen im Abtriebsteil zwischen Zulauf und Seitenabzug und 5 theoretischen Trennstufen zwischen Seitenabzug und Sumpf. Die Kopftemperatur betrug, 118 °C, die Sumpftemperatur 149 °C und die Rücklaufmenge 8.6 kg/h. Über Kopf wurden als Strom (13) 4.1 kg/h EDA mit einem Gehalt von 0.4 % Wasser und 100 ppm Piperazin als Reinprodukt abgezogen (Germisch O). Am Seitenabzug wurden als Strom (14) 0.48 kg/h Piperazin mit einem Gehalt von 98 ppm EDA abgezogen (Gemisch P). Über Sumpf wurden als Strom (15) 50 g/h Rückstand abgezogen (Gemisch Q).

### Stufe b)

Am Sumpf der Kolonne K31 wurden als Strom (16) 8.7 kg/h abgezogen, welcher MEG, DETA und die Schwersieder mit einem Siedepunkt größer oder gleich AEEA enthält (Gemisch C). Dieser Strom wurde in einer Stufe b) in eine Rektifikationskolonne K51 geleitet, wie in Figur 2 dargestellt. Die Kolonne K51 wurde bei einem Kopfdruck von 300 mbar betrieben. Die Kolonne K51 enthielt nur einen Abtriebsteil mit geordneter Packung entsprechend 6 theoretischen Trennstufen. Die Kopftemperatur betrug 161 °C, die Sumpftemperatur 179 °C. Über Kopf wurden als Strom (17) 7.8 kg/h MEG enthaltend 0.4 % DETA und 0.5 % AEEA sowie 0.6 % anderer Hochsieder abgezogen (Gemisch D). Über Sumpf der K51 wurden als Strom (18) 0.91 kg/h gezogen enthaltend 34 % MEG, 34 % DETA, 16 % AEEA und der Rest (16 %) anderer Hochsieder gezogen (Gemisch E).

### Stufe c)

Das Sumpfprodukt aus Stufe b) wurde in einer Stufe c) in eine Rektifikationskolonne K61 geleitet, wie in Figur 2 dargestellt. Die Kolonne K61 wurde bei einem Kopfdruck von 125 mbar betrieben. Die Kolonne K61 enthielt eine geordnete Packung entsprechend 15 theoretischen Trennstufen im Verstärkungs- und 12 theoretischen Trennstufen im Abtriebsteil. Die Kopftemperatur betrug 150 °C, die Sumpftemperatur 180 °C, die Rücklaufmenge 0.8 kg/h. Über Sumpf wurden als Strom (19) 0.29 kg/h Rückstand mit AEEA abgezogen (Gemisch G). Über Kopf wurden als Strom (20) 0.62 kg/h enthaltend ca. 50 % MEG und 50 % DETA mit 110 ppm anderer Hochsieder gezogen (Gemisch F).

### Stufe d)

Der Strom (20) wurde in einer Stufe d) in eine Rektifikationskolonne K71 geleitet, wie in Figur 2 darfestellt. Die Kolonne K71 wurde bei einem Kopfdruck von 30 mbar betrieben. Zusätzlich wurden als Strom (21) 4 kg/h enthaltend hauptsächlich TEG in die K71 geleitet. Die K71 enthielt geordnete Packung entsprechend 6 theoretischen Trennstufen als Verstärkungsteil oberhalb vom Zulauf vom Strom (21), 14 theoretischen Trennstufen zwischen den Zuläufen von Strom (21) (oben) und Strom (20) (unten) sowie 23 theoretischen Trennstufen als Abtriebsteil unterhalb der Zulaufstelle von Strom (20). Die Kopftemperatur betrug 109 °C, die Sumpftemperatur 188 °C und die Rücklaufmenge 0.93 kg/h. Über Kopf der K71 wurden als Strom (22) 0.31 kg/h MEG enthaltend 950 ppm DETA abgezogen (Gemisch H). Strom (22) aus dem Kopf der K71, Strom (17) aus dem Kopf der K51 sowie frisches MEG als Ergänzung der aufgebrauchten Menge wurden vereinigt und als Strom (2) in die Stufe 1 zurückgeführt. Über Sumpf der K71 wurden als Strom (23) 4.3 kg/h abgezogen, welcher DETA und TEG enthält (Gemisch I).

### Stufe 4) TEG-Abtrennung

Der Sumpf der K71 wurde in einer Stufe 4 in eine Rektifikationskolonne K72 eingeleitet, wie in Figur 2 dargestellt. Die Kolonne K72 wurde bei einem Kopfdruck von 25 mbar betrieben. Die Kolonne K72 enthielt geordnete Packung entsprechend 6 theoretischen Trennstufen im Verstärkungs- und 24 theoretischen Trennstufen im Abtriebsteil. Die Kopftemperatur betrug 105 °C, die Sumpftemperatur 190 °C, die Rücklaufmenge 1.4 kg/h. An der K72 wurden als Strom (23) 0.31 kg/h DETA enthaltend 101 ppm MEG und 2500 ppm anderer Hochsieder als Produkt abgezogen (Gemisch J)). Am Sumpf der K72 wurden 4 kg/h, enthaltend TEG, abgezogen (Gemisch K), welche als Strom (21) in die Kolonne K71 in Stufe d) zurückgeführt wurden. Außerdem wurden aus dem Sumpf der K72 10 g/h ausgeschleust, um eine Aufpegelung von inerten Hochsiedern im TEG-Kreislauf zu vermeiden.

### Beispiel 2:

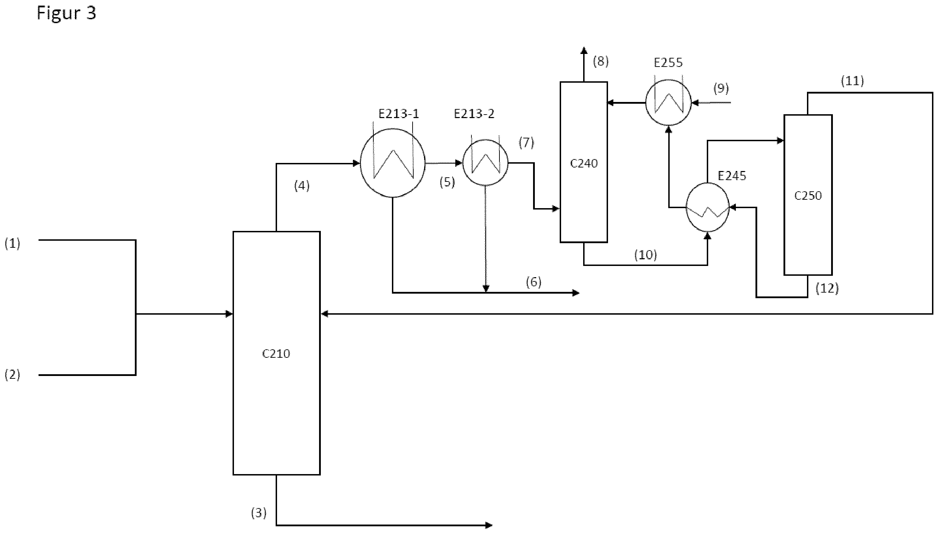

In Beispiel 2 wird die Ammoniakabtrennung (Stufe 2) in mehreren Kolonnen durchgeführt, wie in Figur 3 dargestellt.

Der Austrag aus Stufe 1) (Strom (1)) und Stufe 3 (Strom (2)) wird zusammen in eine Kolonne C210 geleitet, die bei 20 bar betrieben wird.

Die Anzahl der theoretischen Trennstufen beträgt 2. Bei einer Kopftemperatur von ca. 50°C wird ein gasförmiger Strom (Strom (4)) enthaltend Wasserstoff und Ammoniak einem ersten Kondensator zugeführt, in dem das gasförmige Gemisch von 50°C auf 45°C abgekühlt wird. Das gasförmige Gemisch aus dem ersten Kondensator (E213-1) wird in einen zweiten Kondensator

(E213-2) geleitet (Strom (5)), in dem das gasförmige Gemisch auf 35°C abgekühlt wird. Die flüssigen Phasen, welche überwiegend aus Ammoniak bestehen, werden zusammengeführt und als Strom (6) in die Stufe 1) zurückgeführt.

Das Sumpfprodukt aus Kolonne C210 wird in eine weitere Kolonne C220 geleitet, welche 2 theoretische Trennstufen aufweist und bei einem Druck von 20 bar betrieben wird. Am Kopf der Kolonne C220 wird bei einer Kopftemperatur von ca. 49°C ein Strom abgezogen, der überwiegend Ammoniak enthält, der bevorzugt in die Stufe 1 und/oder Stufe 3 zurückgeführt wird. Das Sumpfprodukt aus Kolonne C220 wird in eine weitere Kolonne C230 eingeleitet, die bei 4 bar betrieben wird und 5 theoretische Trennstufen aufweist. Am Kopf der Kolonne C230 wird bei einer Kopftemperatur von ca. 55°C ein Gemisch abgezogen, welches überwiegend Ammoniak und Methylamin enthält. Das Sumpfprodukt wird in die Stufe a) geleitet.

Die gasförmige Phase (Strom (7)) aus dem Kondensator E213-2 wird in eine Waschkolonne C240 geleitet, in der die gasförmige Phase mit Wasser (Strom (9)) im Gegenstrom in Kontakt gebracht wird. Das Waschwasser weist eine Temperatur vo 35°C auf. Das Waschwasser (Strom (9)) ist bevorzugt Wasser, welches in anderen Teilen des Verfahrens als Kühlwasser verwendet worden, beispielsweise zur Kühlung des Kondensators an der EDA-Abtrennung (Stufe 6). Am Kopf der Kolonne C240 wird eine gasförmige Phase abgezogen (Strom (8)), die überwiegend aus Wasserstoff besteht. Das Waschwasser (Strom (10)), welches mit Ammoniak angereichert ist, wird vom Sumpf der Kolonne C240 über einem Wärmetauscher E245 geleitet und dort auf ca. 140°C erwärmt und weiter in eine Kolonne C250 geleitet (Strom (9)). Die Kolonne C250 wird bei einem Druck von 20 bar und einer Sumpftemperatur von 217°C betrieben. Am Kopf der Kolonne C250 wird Ammoniak abgezogen (Strom (11)), welches kondensiert und in die Kolonne C210 zurückgeführt wird. Das von Ammoniak weitestgehend befreite Waschwasser wird in über die Wärmetauscher E245 und E255 in die Kolonne C240 zurückgeführt (Strom (11)). Im Wärmetauscher wird ein Teil der thermischen Energie des Stroms (12) zum Aufheizen des Stroms (10) aus Kolonne C240 genutzt.

### Beispiel 3:

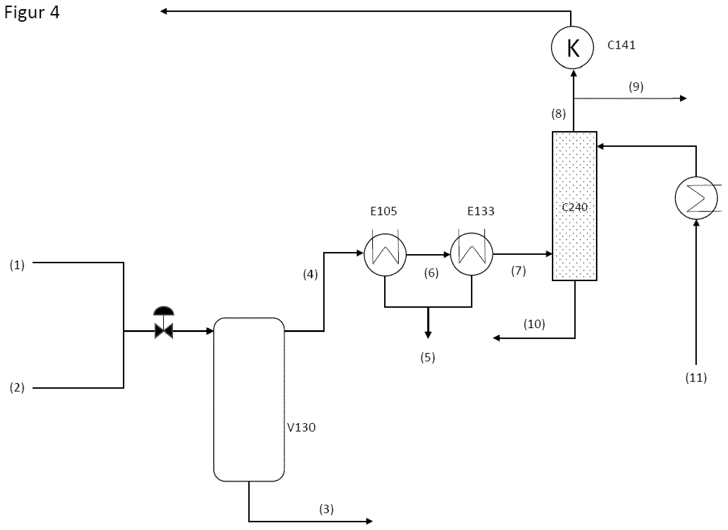

In Beispiel 3 wird die Ammoniakabtrennung in mehreren Kolonnen durchgeführt, wie in Figur 4 dargestellt.

Der Austrag aus Stufe 1) (Strom (1)) und Stufe 3 (Strom (2)) wird zusammengeführt und in über ein Entspannungsventil in einen Flash-Behälter (V130) geleitet. De Flash-Behälter weist einen Druck von 50 bar auf. Die sich bei der Entspannung bildene gasförmige Phase (Strom (4)), welche Wasserstoff und Ammoniak enthält, wird über einen ersten Kondensator (E105) geleitet, wobei die Temperatur der Gasphase von 81°C auf 72°C abgesenkt wird. Das sich bei der Abkühlung bildene Kondensat (Strom (5)), welches überwiegend aus Ammoniak besteht, wird abgezogen und bevorzugt in die Stufe 1 oder 3 zurückgeführt. Die gasförmige Phase aus dem Kondensator E105 wird über einen zweiten Kondensator E133 geleitet (Strom (6)), in dem die Temperatur des Gasstromes von 72°C auf 50°C gesenkt wird. Die sich bildenden Kondensate (Strom (5)) werden mit den flüssigen Kondensaten aus Kondensator E105 vereint und, wie zuvor beschrieben, bevorzugt in die Stufe 1 und/oder Stufe 3 zurückgeführt. Die gasförmige Phase aus Kondensator E133 (Strom (7)) wird in Waschkolonne C240 mit MEG in Kontakt gebracht. In der Waschkolonne C240 wird MEG mit Ammoniak beladen. Das mit Ammoniak beladene MEG (Strom (10)) wird in Stufe 1 und/oder Stufe 3 zurückgeführt. In der Waschkolonne wird das MEG von ca. 46°C auf 91°C erwärmt. Bevorzugt wird als Waschflüssigkeit MEG verwendet, welches vor dem Einleiten in die Stufe 1 in die Waschkolonne C240 eingeleitet wird (Strom (11)), so dass das MEG vor dem Einleiten in Stufe 1 vorgewärmt wird (Strom (10)). Die nicht absorbierte Gasphase aus der Waschkolonne C240, welche überwiegend Wasserstoff enthält (Strom (8)), wird in einem Kompressor C141 auf den in der Stufe 1 herrschenden Reaktionsdruck komprimiert und in die Stufe 1 zurückgeführt. Es ist bevorzugt einen geringen Strom (9) zu entnehmen, um das Aufpegeln von CO und Methan im zurückgeführten Wasserstoff zu vermeiden.

Die flüssige Phase aus Flash-Behälter V130 wird in eine mehrstufige Destillation überführt, in der bei 50 bar zunächst Ammoniak über Kopf abgetrennt wird und das Sumpfprodukt in eine zweite Kolonne geleitet wird, in der bei 4 bar über Kopf weiteres Ammoniak und Methylamin abgetrennt wird. Das Sumpfprodukt aus der zweiten Destillationskolonne wird in die Stufe a) (MEA-Abtrennung) geleitet.

Die in Beispiel 3 aufgeführte Verfahrensvariante hat gegenüber der Verfahrensvariante aus Beispiel 2 den Vorteil, dass als Waschflüssigkeit ein Edukt verwendet wird, welches ohne weitere Aufreinigung in die MEG-Umsetzung (Stufe 1) eingesetzt werden kann. Das Waschwasser aus Beispiel 2 muss hingegen in weiteren Verfahrensschritten aufgearbeitet werden. Weiterhin wurde überraschender Weise gefunden, dass sich die Waschflüssigkeit MEG beim Inkontaktbringen mit Ammoniak in Kolonne C240 erwärmt. So kann MEG vor dem Einleiten in die MEG-Umsetzung (Stufe 1) bereits aufgewärmt werden, so dass zum Erwärmen der Produkte in Stufe 1 weniger Energie benötigt wird. wird in einem Kompressor C141 auf den in der Stufe 1 herrschenden Reaktionsdruck komprimiert und in die Stufe 1 zurückgeführt. Es ist bevorzugt einen geringen Strom (9) zu entnehmen, um das Aufpegeln von CO und Methan im zurückgeführten Wasserstoff zu vermeiden.

Die flüssige Phase aus Flash-Behälter V130 wird in eine mehrstufige Destillation überführt, in der bei 50 bar zunächst Ammoniak über Kopf abgetrennt wird und das Sumpfprodukt in eine zweite Kolonne geleitet wird, in der bei 4 bar über Kopf weiteres Ammoniak und Methylamin abgetrennt wird. Das Sumpfprodukt aus der zweiten Destillationskolonne wird in die Stufe a) (MEA-Abtrennung) geleitet.

Die in Beispiel 3 aufgeführte Verfahrensvariante hat gegenüber der Verfahrensvariante aus Beispiel 2 den Vorteil, dass als Waschflüssigkeit ein Edukt verwendet wird, welches ohne weitere Aufreinigung in die MEG-Umsetzung (Stufe 1) eingesetzt werden kann. Das Waschwasser aus Beispiel 2 muss hingegen in weiteren Verfahrensschritten aufgearbeitet werden. Weiterhin wurde überraschender Weise gefunden, dass sich die Waschflüssigkeit MEG beim Inkontaktbringen mit Ammoniak in Kolonne C240 erwärmt. So kann MEG vor dem Einleiten in die MEG-Umsetzung (Stufe 1) bereits aufgewärmt werden, so dass zum Erwärmen der Produkte in Stufe 1 weniger Energie benötigt wird.

## Patentansprüche

1. Verfahren zur Herstellung von Ethylenaminen und/oder Alkanolaminen welches folgende Schritte umfasst:
1) Umsetzung von MEG mit Ammoniak in Gegenwart von Wasserstoff und eines Aminierungskatalysators;
2) Abtrennung von Wasserstoff und Ammoniak aus dem Reaktionsaustrag aus Stufe 1, **dadurch gekennzeichnet, dass** die Abtrennung von Wasserstoff und Ammoniak in Stufe 2 folgende Schritte umfasst:
2-1) Auftrennung des Reaktionsaustrags aus Stufe 1 in eine gasförmige Phase, welche Ammoniak und Wasserstoff enthält, und eine flüssige Phase, welche Ethylenamine und/oder Alkanolamine enthält,
2-2) Überleitung der gasförmigen Phase aus Stufe 2-1) über ein oder mehrere Kondensatoren, wobei eine oder mehrere flüssige Phase erhalten werden, in denen Ammoniak angereichert ist, und eine gasförmige Phase erhalten wird, in der Wasserstoff angereichert ist,
2-3) Inkontaktbringen der gasförmigen Phase aus Stufe 2-2) mit MEG, so dass eine flüssige Phase, enthaltend MEG und Ammoniak, erhalten wird und eine gasförmige Phase, enthaltend Wasserstoff und optional Ammoniak, erhalten wird.

2. Verfahren nach Anspruch 1, **dadurch gekennzeichnet, dass** das MEG vor dem Inkontaktbringen mit der gasförmigen Phase aus Stufe 2-2) eine Temperatur im Bereich von 20 bis 80°C aufweist.

3. Verfahren nach mindestens einem der Ansprüche 1 bis 2, **dadurch gekennzeichnet, dass** das MEG, welches in Stufe 2-3 verwendet wird einen Gehalt von Schwefel von weniger als 100 ppm enthält.

4. Verfahren nach mindestens einem der Ansprüche 1 bis 3, **dadurch gekennzeichnet, dass** die flüssige Phase aus Stufe 2-3 in Stufe 1) geleitet wird.

5. Verfahren nach mindestens einem der Ansprüche 1 bis 4, **dadurch gekennzeichnet, dass** die Stufe 2-1 bei einem Druck im Bereich von 20 bis 80 bar erfolgt.

6. Verfahren nach mindestens einem der Ansprüche 1 bis 5, **dadurch gekennzeichnet, dass** die Stufe 2-2) aus einem Kondensator besteht.

7. Verfahren nach mindestens einem der Ansprüche 1 bis 5, **dadurch gekennzeichnet, dass** die Stufe 2-2) aus zwei Kondensatoren besteht.

8. Verfahren nach mindestens einem der Ansprüche 1 bis 7, **dadurch gekennzeichnet, dass** die ein oder mehreren Kondensatoren in Stufe 2-2) so ausgelegt sind, dass die Temperatur der gasförmigen Phase aus Stufe 2-2), die in Stufe 2.3) eingeleitet wird, im Bereich von 25 bis 75°C liegt.

9. Verfahren nach mindestens einem der Ansprüche 1 bis 8, **dadurch gekennzeichnet, dass** die flüssigen Phasen aus Stufe 2-2) getrennt oder zusammen in die Stufe 1) zurückgeführt werden.

10. Verfahren nach mindestens einem der Ansprüche 1 bis 9, **dadurch gekennzeichnet, dass** die gasförmige Phase aus Stufe 2-3) komprimiert wird und in die Stufe 1) zurückgeführt wird.

11. Verfahren nach mindestens einem der Ansprüche 1 bis 10, **dadurch gekennzeichnet, dass** die Stufe 1) bei einem Druck im Bereich von 50 bis 500 bar durchgeführt wird.

12. Verfahren nach mindestens einem der Ansprüche 1 bis 11, **dadurch gekennzeichnet, dass** die Stufe 1) bei einer Temperatur im Bereich von 50 bis 350°C durchgeführt wird.

## Claims

1. A process for preparing ethyleneamines and/or alkanolamines, comprising the following steps:
1) reacting MEG with ammonia in the presence of hydrogen and an amination catalyst;
2) removing hydrogen and ammonia from the reaction output from stage 1,
wherein the removal of hydrogen and ammonia in stage 2 comprises the following steps:
2-1) separating the reaction output from stage 1 into a gaseous phase comprising ammonia and hydrogen, and a liquid phase comprising ethyleneamines and/or alkanolamines,
2-2) passing the gaseous phase from stage 2-1) through one or more condensers to obtain one or more liquid phases in which ammonia has been enriched, and a gaseous phase in which hydrogen has been enriched,
2-3) contacting the gaseous phase from stage 2-2) with MEG so as to obtain a liquid phase comprising MEG and ammonia and a gaseous phase comprising hydrogen and optionally ammonia.

2. The process according to claim 1, wherein the MEG before being contacted with the gaseous phase from stage 2-2) is at a temperature in the range from 20 to 80°C.

3. The process according to at least one of claims 1 and 2, wherein the MEG which is used in stage 2-3 comprises a sulfur content of less than 100 ppm.

4. The process according to at least one of claims 1 to 3, wherein the liquid phase from stage 2-3 is passed into stage 1).

5. The process according to at least one of claims 1 to 4, wherein stage 2-1 is effected at a pressure in the range from 20 to 80 bar.

6. The process according to at least one of claims 1 to 5, wherein stage 2-2) consists of one condenser.

7. The process according to at least one of claims 1 to 5, wherein stage 2-2) consists of two condensers.

8. The process according to at least one of claims 1 to 7, wherein the one or more condensers in stage 2-2) are designed such that the temperature of the gaseous phase from stage 2-2) which is introduced into stage 2.3) is in the range from 25 to 75°C.

9. The process according to at least one of claims 1 to 8, wherein the liquid phases from stage 2-2) are recycled separately or together into stage 1).

10. The process according to at least one of claims 1 to 9, wherein the gaseous phase from stage 2-3) is compressed and recycled into stage 1).

11. The process according to at least one of claims 1 to 10, wherein stage 1) is conducted at a pressure in the range from 50 to 500 bar.

12. The process according to at least one of claims 1 to 11, wherein stage 1) is conducted at a temperature in the range from 50 to 350°C.

## Revendications

1. Procédé de préparation d'éthylèneamines et/ou d'alcanolamines, qui comprend les étapes suivantes :
1) réaction de MEG avec de l'ammoniac en présence d'hydrogène et d'un catalyseur d'amination ;
2) séparation de l'hydrogène et de l'ammoniac d'avec le produit de la réaction de l'étape 1, **caractérisé en ce que** la séparation de l'hydrogène et de l'ammoniac dans l'étape 2 comprend les étapes suivantes :
2-1) séparation du produit de la réaction de l'étape 1 en une phase gazeuse, qui contient l'ammoniac et l'hydrogène, et une phase liquide, qui contient les éthylèneamines et/ou les alcanolamines,
2-2) transfert de la phase aqueuse de l'étape 2-1) sur un ou plusieurs condensateurs, avec obtention d'une ou plusieurs phases liquides, dans lesquelles l'ammoniac est enrichi, et avec obtention d'une phase gazeuse, dans laquelle l'hydrogène est enrichi,
2-3) mise en contact de la phase gazeuse de l'étape 2-2) avec du MEG, avec obtention d'une phase liquide contenant le MEG et l'ammoniac, et obtention d'une phase gazeuse contenant de l'hydrogène et éventuellement de l'ammoniac.

2. Procédé selon la revendication 1, **caractérisé en ce que** le MEG, avant la mise en contact avec la phase gazeuse de l'étape 2-2), présente une température dans la plage de 20 à 80 °C.

3. Procédé selon au moins l'une des revendications 1 à 2, **caractérisé en ce que** le MEG qui est utilisé dans l'étape 2-3 contient du soufre en une quantité inférieure à 100 ppm.

4. Procédé selon au moins l'une des revendications 1 à 3, **caractérisé en ce que** la phase liquide de l'étape 2-3 est envoyée dans l'étape 1).

5. Procédé selon au moins l'une des revendications 1 à 4, **caractérisé en ce que** l'étape 2-1 est mise en œuvre sous une pression dans la plage de 20 à 80 bar.

6. Procédé selon au moins l'une des revendications 1 à 5, **caractérisé en ce que** l'étape 2-2) est constituée d'un condensateur.

7. Procédé selon au moins l'une des revendications 1 à 5, **caractérisé en ce que** l'étape 2-2) est constituée de deux condensateurs.

8. Procédé selon au moins l'une des revendications 1 à 7, **caractérisé en ce que** le ou les condensateurs de l'étape 2-2) sont agencés de telle sorte que la température de la phase gazeuse de l'étape 2-2), qui est envoyée dans l'étape 2-3), soit comprise dans la plage de 25 à 75 °C.

9. Procédé selon au moins l'une des revendications 1 à 8, **caractérisé en ce que** les phases liquides de l'étape 2-2) sont renvoyées dans l'étape 1), séparément ou ensemble.

10. Procédé selon au moins l'une des revendications 1 à 9, **caractérisé en ce que** la phase gazeuse de l'étape 2-3) est comprimée et renvoyée dans l'étape 1).

11. Procédé selon au moins l'une des revendications 1 à 10, **caractérisé en ce que** l'étape 1) est mise en œuvre sous une pression dans la plage de 50 à 500 bar.

12. Procédé selon au moins l'une des revendications 1 à 11, **caractérisé en ce que** l'étape 1) est mise en œuvre à une température dans la plage de 50 à 350 °C.
